# EUROPEAN PATENT APPLICATION

(11) **EP 4 843 490 A2**
(43) Date of publication of application: **25.11.2026**
(21) Application number: 26211240.2
(22) Date of filing: 02.12.2022
(51) Int. Cl.: A61B 5/03

(54) **BRAIN WATER MONITORING METHOD AND APPARATUS BASED ON NEAR-INFRARED SPECTROSCOPY**

(30) Priority: 03.12.2021 KR 20210171613; 01.12.2022 KR 20220166047
(62) Document number(s) of the earlier application(s) in accordance with Art. 76 EPC: 22901864.3 / 4 442 191
(71) Applicant: Seoul National University Hospital, Seoul 03080 (KR)
(72) Inventor: YUN, Chang-Ho, 13620 Seongnam-si (KR); YOON, Jee-Eun, 13620 Seongnam-si (KR); JO, You Hwan, 13620 Seongnam-si (KR); KANG, Jihoon, 13620 Seongnam-si (KR); KIM, Jun Yup, 13620 Seongnam-si (KR)
(74) Representative: V.O.

(57) **Abstract**

The objective of the present invention is to provide a brain water monitoring method and apparatus based on near-infrared spectroscopy, which can non-invasively and accurately measure the brain water activity of brain tissue according to sleep-wakefulness and a sleep structure on the basis of near-infrared spectroscopy without injecting a contrast agent, an aqueous solution, or the like to the human body (intravenously or intrathecally). To achieve the objective, the present invention comprises: calculating oxidized hemoglobin, reduced hemoglobin, and water fraction based on signals measured by near-infrared spectroscopy; estimating a hemoglobin-correlated component included in the water fraction by using linear estimation based on a correlation between the water fraction and a hemoglobin signal; extracting a pure water fraction from the water fraction by removing the estimated hemoglobin-correlated component; and outputting the extracted pure water fraction.

## Description

### Filed of the Invention

The present invention relates to a method and device for monitoring brain water based on near-infrared spectroscopy. More specifically, the present invention relates to a method and device for monitoring brain water based on near-infrared spectroscopy (NIRS) that allow quantitative measurement and monitoring of glymphatic system activity, brain edema, and intracranial pressure increase, etc. by measuring the amount of brain water in a real-time, non-invasive manner based on near-infrared spectroscopy.

### [National research and development project that supported this invention]

[Assignment number] 1711163115
[Assignment number] 2019R1A2C2086705
[Ministry name] Ministry of Science and ICT
[Name of project management (professional) organization] National Research Foundation of Korea
[Research project name] Individual basic research (Ministry of Science and ICT) (R&D)
[Research project title] Brain disease prediction and intervention research through the development of a brain washing mechanism (glymphatic system) observation system
[Contribution rate] 1/1
[Name of project carrying out organization] Korea Advanced Institute of Science and Technology
[Research period] 2019.09.01 ~ 2023.08.31

### Background of the Invention

Recently, it was discovered that the central nervous system also has a pathway that excretes metabolites from brain cells to maintain stable function of brain cells, and this was named the 'glymphatic system'.

In the glymphatic system, cerebrospinal fluid (CSF) moves along the perivascular space and permeates into the brain parenchyma through aquaporin-4 (AQP-4) water channels expressed at the terminals of astrocytes surrounding the cerebral vasculature. It is a system in which waste products are removed through solute exchange by mixing with interstitial fluid.

High brain activity, which accounts for 20% of body metabolism, is accompanied by the production of many waste products, including abnormal proteins, so appropriate glymphatic system activity is very important for brain health.

In particular, the glymphatic system plays an important role in removing pathological proteins such as amyloid beta and tau protein from the brain parenchyma, which are the causes of Alzheimer's disease.

Therefore, decreased or abnormal function of the glymphatic system prevents interstitial wastes, including amyloid beta and tau protein, from being properly removed, increasing the risk of developing neurodegenerative diseases such as Alzheimer's disease, Parkinson's disease, amyotrophic lateral sclerosis, and Huntington's disease.

It is known that the activity of this glymphatic system increases by more than 60% during sleep and is suppressed upon awakening.

Considering the important role of the glymphatic system in normal brain function and pathophysiology, objective and quantitative measurement of glymphatic system activity is necessary.

As a method to quantitatively measure the existing glymphatic system, there is a method using two-photon microscopy.

Two-photon microscopy, a quantitative glymphatic system measurement technique used in animal experiments, measures glymphatic system activity by checking the movement and distribution of fluorescent substances injected into cerebrospinal fluid, but is invasive and is not suitable for application to the human body.

To overcome the shortcomings of the two-photon microscopy technique, there has recently been an attempt to identify the glymphatic system pathway by administering gadobutrol contrast media into the human cerebrospinal fluid and then repeatedly taking brain magnetic resonance imaging (MRI). There are disadvantages that intrathecal administration of gadobutrol is not only not FDA-approved, but also that it is difficult to measure continuously in real time, and that it is impossible to track dynamic changes according to wakefulness-sleep transition and sleep stages, and that the measurement disrupts natural sleep.

Meanwhile, stroke patients may initially experience rapid changes in neurological function due to rapid changes in blood pressure, blood sugar, electrolytes, fever, etc. Deterioration of early neurological function occurs in 30-40% of acute stroke patients, and has a significant impact on long-term neurological prognosis and mortality.

In particular, after vascular revascularization treatment for acute ischemic stroke, cerebral blood flow may not be sufficiently supplied or re-occlusion may occur, and hemorrhagic transformation may occur due to excessive blood supply, so monitoring and diagnosis of early neurological function deterioration is very important.

However, to date, there is no technology to monitor the deterioration of early neurological function in real time, and the only methods available are close clinical observation by medical staff in a stroke intensive care unit or neurological intensive care unit and imaging tests using intermittent CT or MRI.

CT or MRI imaging tests have limitations that they take time to complete, there are concerns about patient safety issues due to spatial distance and transportation, and they are intermittent evaluations of brain conditions. Considering the possibility of rapid changes in various acute brain diseases, non-invasive continuous monitoring equipment is needed.

Diagnosis of cerebral edema, which can occur in various diseases such as cerebral infarction, cerebral hemorrhage, head trauma, brain tumor, and encephalitis, currently uses CT or MRI imaging tests.

However, in most cases, it is clinically confirmed after rapid cerebral edema progresses, often resulting in irreversible brain damage.

In addition, patients with cerebral edema are usually in a severe neurological condition and have restrictions on movement due to the possibility of neurological changes and deterioration of vital signs during transport, making it difficult to repeat imaging tests.

Since the change in cerebral pressure due to cerebral edema is measured by inserting a tube into the cerebrospinal fluid, it is not only invasive, but there may also be complications such as concerns about infection and brain herniation during the drainage tube insertion process.

### [Prior art literature]

US2016/0310054 A1

### Detailed Description of the Invention

### Technical Challenges

The present invention was developed to solve the conventional problems as described above, and the purpose is to provide a brain water monitoring method and device based on near-infrared spectroscopy that can accurately measure non-invasively the brain activity of brain tissue depending on sleep-wake and sleep structure based on near-infrared spectroscopy without injecting contrast media or aqueous solutions into the human body (venous or intrathecal).

Another object of the present invention is to provide a method and device for monitoring and quantitatively measuring the glymphatic system activity based on the measured brain water activity of brain tissue.

Another object of the present invention is to provide a method and device that can more precisely evaluate the state of sleep in more granular stages based on the measured brain water activity or glymphatic system activity.

Another object of the present invention is to provide a method and device for providing information on whether the measured glymphatic system activity is normal or abnormal by providing standard data values that can be determined to be in the normal range.

Another object of the present invention is to provide methods and devices that provide information on health indicators (which may include lifestyle correction factors), diagnosis and/or progression, pathophysiology (including occurrence mechanisms and causes), and/or treatment effects of normal aging and neurodegenerative brain diseases such as Alzheimer's disease and Parkinson's disease or other neurological diseases based on measured glymphatic system activity.

Another object of the present invention is a method and device for monitoring and/or quantitatively measuring cerebral edema or increased intracranial pressure accompanying brain diseases such as cerebral infarction, cerebral hemorrhage, head trauma, brain tumor, and encephalitis, based on the measured brain water activity of brain tissue.

Another object of the present invention is to provide a method and device for controlling a cerebral pressure regulator such as a shunt based on the measured brain water activity of brain tissue.

### Technical Solution

A method for monitoring brain water based on near-infrared spectroscopy according to the present invention for achieving the above-described object is characterized by comprising: a calculation step of calculating oxidized hemoglobin, reduced hemoglobin, and water fraction based on signals measured by near-infrared spectroscopy; an extraction step of extracting a pure water fraction from the water fraction calculated in the calculation step; and an ouput step of outputting the water fraction extracted in the extraction step.

In addition, the method for monitoring brain water based on near-infrared spectroscopy according to the present invention is characterized by further comprising a removal step of removing motion artifacts from the signal measured using the near-infrared spectroscopy, wherein the removal step of is performed before or after the calculation step.

In addition, in the method for monitoring brain water based on near-infrared spectroscopy according to the present invention, the removal step is characterized by removing motion artifacts by low-pass filtering the signal measured using the near-infrared spectroscopy and using spline interpolation.

In addition, in the method for monitoring brain water based on near-infrared spectroscopy according to the present invention, the extraction step is characterized by extracting a pure water fraction by removing the oxidized hemoglobin fraction and the reduced hemoglobin fraction from the water fraction calculated in the calculation step.

In addition, in the method for monitoring brain water based on near-infrared spectroscopy according to the present invention, the extraction step of extracting is characterized by removing the oxidized hemoglobin fraction and reduced hemoglobin fraction from the water fraction calculated in the calculation step, but extracting a pure water fraction by removing the oxidized hemoglobin fraction and reduced hemoglobin fraction from the water fraction calculated using Linear Minimum Mean Square Error (LMMSE).

In addition, a method for measuring glymphatic system activity according to the present invention for achieving the above-described object is characterized by comprising: a calculation step of calculating oxidized hemoglobin, reduced hemoglobin, and water fraction based on signals measured by near-infrared spectroscopy; an extraction step of extracting a pure water fraction from the water fraction calculated in the calculation step; and an information provision step of providing information on glymphatic system activity based on the water fraction extracted in the extraction step.

In addition, in the method for measuring glymphatic system activity according to the present invention, the information provision step is characterized by measuring glymphatic system activity based on the water fraction in the extraction step and providing information on the measured glymphatic system activity.

In addition, a method for evaluating glymphatic system activity according to the present invention for achieving the above-described object is characterized by comprising: a standard data construction step of constructing standard data of brain tissue water based on measurements taken in the selected group; a calculation step of calculating oxidized hemoglobin, reduced hemoglobin, and water fraction based on signals measured by near-infrared spectroscopy; an extraction step of extracting a pure water fraction from the water fraction calculated in the calculation step; and an information provision step of providing information comprising evaluating glymphatic system activity based on the water fraction extracted in the extraction step and the standard data of brain tissue water and providing evaluation information.

In addition, in the method for evaluating glymphatic system activity according to the present invention, the information provision step is characterized by comparing the water fraction extracted in the extraction step with the standard data of brain tissue water, and if a significant difference occurs with the standard data of brain tissue water, providing information by scoring it with a categorization result or standardized value corresponding to the difference.

In addition, a method for evaluating sleep state according to the present invention for achieving the above-described object is characterized by comprising: a standard data construction step of constructing standard data of brain tissue water based on measurements taken in the selected group; a calculation step of calculating oxidized hemoglobin, reduced hemoglobin, and water fraction based on signals measured by near-infrared spectroscopy; an extraction step of extracting a pure water fraction from the water fraction calculated in the calculation step; and an information provision step of providing information on the sleep state according to glymphatic system activity in wakefulness-sleep or sleep stages based on the water fraction extracted in the extraction step and the standard data of brain tissue water.

In addition, in the method for evaluating sleep state according to the present invention, the information provision step is characterized by comparing the water fraction extracted in the extraction step with the standard data of brain tissue water, and if a significant difference occurs with the standard data of brain tissue water, providing information on the sleep state according to glymphatic system activity by scoring it with a categorization result or standardized value corresponding to the difference.

In addition, in the method for evaluating sleep state according to the present invention, the information provision step is characterized by providing information on the sleep state according to glymphatic system activity by monitoring changes in the water fraction according to sleep stage transition based on the water fraction extracted in the extraction step.

In addition, a method for providing auxiliary information related to neurodegenerative brain disease according to the present invention for achieving the above-described object is characterized by comprising: a database (DB) construction step of constructing database comprising neurodegenerative brain disease indicators corresponding to glymphatic system activity; a calculation step of calculating oxidized hemoglobin, reduced hemoglobin, and water fraction based on signals measured by near-infrared spectroscopy; an extraction step of extracting a pure water fraction from the water fraction calculated in the calculation step; and an information provision step of providing auxiliary information related to neurodegenerative brain disease based on the water fraction extracted in the extraction step and the database.

In addition, in the method for providing auxiliary information related to neurodegenerative brain disease according to the present invention, the information provision step is characterized by measuring glymphatic system activity based on the water fraction extracted in the extraction step, searching a neurodegenerative brain disease indicator corresponding to the measured the glymphatic system activity in the database, and then providing auxiliary information related to prediction, progression, diagnosis, and treatment of neurodegenerative brain disease based on the searched indicator.

In addition, in the method for providing auxiliary information related to neurodegenerative brain disease according to the present invention, the DB construction step constructs standard data of brain tissue water, and the information provision step is characterized by comparing the water fraction extracted in the extraction step with the standard data of brain tissue water, and if a significant difference occurs with the standard data of brain tissue water, providing auxiliary information related to prediction, progression, diagnosis, and treatment of neurodegenerative brain disease by scoring it with a categorization result or standardized value corresponding to the difference.

In addition, a method for measuring cerebral edema and cerebral pressure according to the present invention for achieving the above-described object is characterized by comprising: a calculation step of calculating oxidized hemoglobin, reduced hemoglobin, and water fraction based on signals measured by near-infrared spectroscopy; an extraction step of extracting a pure water fraction from the water fraction calculated in the calculation step; and an information provision step of providing information comprising measuring cerebral edema and cerebral pressure based on the water fraction extracted in the extraction step and providing information on the measured cerebral edema and cerebral pressure.

In addition, in the method for measuring cerebral edema and cerebral pressure according to the present invention, the information provision step is characterized by measuring water amount of brain tissue at regular intervals, comparing it with the initial value, and providing information on the cerebral edema and cerebral pressure by scoring the difference with a categorization result or standardized value corresponding to the difference.

In addition, in the method for measuring cerebral edema and cerebral pressure according to the present invention, the method is characterized by further comprising: a control step of controlling the cerebral pressure regulator based on the cerebral pressure information measured in the providing information.

In addition, a device for monitoring brain water based on near-infrared spectroscopy according to the present invention for achieving the above-described object comprises: a near-infrared spectrometer unit that outputs a near-infrared signal to a subject and receives a signal reflected from the subject; and a data processing unit for processing the signal obtained from the near-infrared spectrometer unit, wherein the data processing unit is characterized in monitoring brain water changes by performing the near-infrared spectroscopy-based brain water monitoring method according to the present invention.

In addition, a device for measuring glymphatic system activity according to the present invention for achieving the above-described object comprises: a near-infrared spectrometer unit that outputs a near-infrared signal to a subject and receives a signal reflected from the subject; and a data processing unit for processing the signal obtained from the near-infrared spectrometer unit, wherein the data processing unit is characterized by measuring glymphatic system activity by performing the method for measuring glymphatic system activity according to the present invention.

In addition, a device for evaluating glymphatic system activity according to the present invention for achieving the above-described object comprises: a near-infrared spectrometer unit that outputs a near-infrared signal to a subject and receives a signal reflected from the subject; and a data processing unit for processing the signal obtained from the near-infrared spectrometer unit, wherein the data processing unit is characterized by comprising standard data of brain tissue water and evaluating glymphatic system activity by performing the method for evaluating glymphatic system activity according to the present invention.

In addition, a device for evaluating sleep state according to the present invention for achieving the above-described object comprises: a near-infrared spectrometer unit that outputs a near-infrared signal to a subject and receives a signal reflected from the subject; and a data processing unit for processing the signal obtained from the near-infrared spectrometer unit, wherein the data processing unit is characterized by providing information on the sleep state according to glymphatic system activity in wakefulness-sleep or sleep stages by performing the method for evaluating sleep state according to the present invention.

In addition, a device for providing auxiliary information related to neurodegenerative brain disease according to the present invention for achieving the above-described object comprises: a near-infrared spectrometer unit that outputs a near-infrared signal to a subject and receives a signal reflected from the subject; a data processing unit for processing the signal obtained from the near-infrared spectrometer unit; and database comprising neurodegenerative brain disease indicators corresponding to glymphatic system activity, wherein the data processing unit is characterized by providing auxiliary information related to neurodegenerative brain disease by performing the method for providing auxiliary information related to neurodegenerative brain disease according to the present invention.

In addition, a device for measuring cerebral edema and cerebral pressure according to the present invention for achieving the above-described object comprises: a near-infrared spectrometer unit that outputs a near-infrared signal to a subject and receives a signal reflected from the subject; and a data processing unit for processing the signal obtained from the near-infrared spectrometer unit, wherein the data processing unit is characterized by providing information on the measured cerebral edema and cerebral pressure by performing the method for measuring cerebral edema and cerebral pressure according to the present invention.

In addition, in the device for measuring cerebral edema and cerebral pressure according to the present invention, wherein the device is characterized by further comprising a control unit to control the cerebral pressure regulator based on the cerebral pressure output from the data processing unit.

Specific details of other embodiments are included in "Specific Details for Carrying Out the Invention" and the attached "Drawings."

The advantages and/or features of the present invention and methods for achieving them will become clear by referring to the various embodiments described in detail below along with the accompanying drawings.

However, the present invention is not limited to the configuration of each embodiment disclosed below, but may also be implemented in various different forms. However, each embodiment disclosed in this specification ensures that the disclosure of the present invention is complete, and is provided to fully inform those skilled in the art of the scope of the present invention, and it should be noted that the present invention is only defined by the scope of each claim in the claims.

### Effects of the Invention

In the case of methods such as a measurement method of the glymphatic system of prior technologies, there are disadvantages that it is impossible or difficult to apply to the human body due to invasive methods, real-time continuous measurement is difficult, continuous evaluation of changes in glymphatic system activity according to the transition between wakefulness and sleep is impossible, and the complexity of the measurement process disrupts natural sleep.

In comparison, the method and device of exemplary embodiments of the present invention can be applied to the human body non-invasively and continuously measure in real time, providing the advantage of not having significant limitations in conditions such as wakefulness and sleep.

In addition, in exemplary embodiments of the present invention, since it is important for signal analysis to remove various artifacts that occur in the process of measuring biosignals, the quality of analysis can be improved removing all motion artifacts of heart rate, respiration, pulse, and movement, and brain water activity can be measured more accurately. In addition, the precision of extracting the pure water value signal can be further increased by performing the process of extracting the pure water value together after removing the motion artifact.

In particular, it is necessary to obtain pure water in order to measure brain water activity more accurately, rather than simply confirming changes in water absorption. Here, it is important to resolve cross-talk artifacts, and to resolve this, embodiments of the present invention used, for example, ordinary least square regression analysis applying LMMSE.

Moreover, prior research to date has mainly confirmed changes in the glymphatic system activity according to the transition between wakefulness and sleep. However, exemplary embodiments of the present invention can provide the advantage of more precisely monitoring activity changes not only according to the transition between wakefulness and sleep but also according to sleep stages, even activity changes in NREM sleep at the beginning and end of sleep. Accordingly, sleep state can be monitored more precisely and used for diagnosis or treatment.

In addition, according to exemplary embodiments of the present invention, useful auxiliary information can be provided for health indicators, prediction, diagnosis, and treatment of normal aging and degenerative brain diseases such as Alzheimer's disease and Parkinson's disease based on glymphatic system activity, and can be used as a key element of pathophysiology.

In addition, based on the brain water activity of brain tissue, changes in cerebral edema and intracranial pressure increasement in brain diseases such as cerebral infarction, cerebral hemorrhage, head trauma, brain tumor, and encephalitis can be noninvasively measured and/or monitored, and invasive procedures can also be minimized when controlling the intracranial pressure regulator such as shunts.

Accordingly, it is possible to secure the ease and speed of prediction, diagnosis, and treatment of various brain-related diseases such as severe neurological diseases in emergency treatment settings or hospitals. It is also possible to realize the improvement of the treatment prognosis through preemptive response through early detection of changes in the state of the brain disease, solving unmet medical needs that have no choice but to rely on existing neuroimaging (CT, MRI, etc.) and replace or minimize/optimize the performance of imaging tests.

### Brief Description of the Drawings

FIG. 1 is a processing diagram for explaining a brain water monitoring method based on near-infrared spectroscopy according to an embodiment of the present invention.
FIG. 2 is a diagram schematically showing the configuration of a device for monitoring brain water based on a near-infrared spectroscopy according to an embodiment of the present invention.
FIG. 3 is a processing diagram for explaining a method for measuring the glymphatic system activity according to an embodiment of the present invention.
FIG. 4 is a diagram schematically showing the configuration of a device for measuring the glymphatic system activity according to an embodiment of the present invention.
FIG. 5 is a processing diagram for explaining a method for evaluating the glymphatic system activity according to an embodiment of the present invention.
FIG. 6 is a diagram schematically showing the configuration of a device for evaluating the glymphatic system activity according to an embodiment of the present invention.
FIG. 7 is a processing diagram for explaining a method for evaluating sleep state according to an embodiment of the present invention.
FIG. 8 is a diagram schematically showing the configuration of a device for evaluating sleep state according to an embodiment of the present invention.
FIG. 9 is a processing diagram for explaining a method of providing auxiliary information related to neurodegenerative brain disease according to an embodiment of the present invention.
FIG. 10 is a diagram schematically showing the configuration of a device for providing auxiliary information related to neurodegenerative brain disease according to an embodiment of the present invention.
FIG. 11 is a processing diagram for explaining a method of measuring intracranial pressure according to an embodiment of the present invention.
FIG. 12 is a diagram schematically showing the configuration of a device for measuring intracranial pressure according to an embodiment of the present invention.
FIG. 13 is a diagram showing an exemplary installation state of polysomnography (PSG) and NIRS probes.
FIG. 14 is a diagram showing an exemplary NIRS device applied to this experiment.
FIG. 15 is a diagram showing an exemplary NIRS measurement optical path.
FIG. 16 is a diagram showing time series data during sleep stage transition.
FIG. 17 is a diagram showing a boxplot of water fraction change over time quantized in 5-minute increments.
FIG. 18 is a diagram showing the average water percentage after each sleep transition.
FIG. 19 is a diagram showing the comparison of water fraction change between the first NREM stage and the last NREM stage.
FIG. 20 is a diagram showing the average water content of each REM stage.

### Best Mode for Carrying Out the Invention

Before explaining the present invention in detail, the terms or words used in this specification should not be construed as unconditionally limited to their ordinary or dictionary meanings. It should be noted that in order to explain the invention in the best way, the inventor of the present invention can appropriately define and use the concepts of various terms, and further interpret these terms and words with meanings and concepts consistent with the technical idea of the present invention.

That is, it should be noted that the terms used in this specification are only used to describe preferred embodiments of the present invention, and are not used with the intention of specifically limiting the content of the present invention, and these terms are defined in consideration of various possibilities of the present invention.

In addition, in this specification, it should be noted that singular expressions may include plural expressions unless the context clearly indicates a different meaning, and that even if similarly expressed in plural, they may include singular meanings.

Throughout this specification, when a component is described as "including" another component, it could mean that it does not exclude any other component, but rather may include any other component, unless specifically stated to the contrary.

Furthermore, if a component is described as being "present within or installed in contact with" another component, it should be noted that this component may be installed directly connected to or in contact with another component, or may be installed at a certain distance, and if the component is installed at a certain distance, there may be a third component or means to fix or connect the component to another component, and description of this third component or means may be omitted.

On the other hand, when a component is described as being "directly connected" or "directly connected" to another component, it should be understood that no third component or means is present.

Likewise, other expressions that describe the relationship between each component, such as "between" and "immediately between", or "neighboring" and "directly neighboring", should be interpreted as the same meaning.

In addition, in this specification, terms such as "one side", "other side", "one side", "the other side", "first", "second", etc., if used, are used for one component so that this one component can be clearly distinguished from other components, and it should be noted that the meaning of the corresponding component is not limited by such term.

In addition, in this specification, terms related to position such as "top", "bottom", "left", "right", etc., if used, should be understood as indicating the relative position of the corresponding component in the corresponding drawing. Unless the absolute location is specified, these location-related terms should not be understood as referring to the absolute location.

Moreover, in the specification of the present invention, term such as "... unit", "... unit", "module", "device", etc., when used, mean a unit capable of processing one or more functions or operations, and it should be noted that it can be implemented through hardware, software, or a combination of hardware and software.

In addition, in this specification, when specifying the reference numeral for each component in each drawing, the same component has the same reference number even if the component is shown in different drawings, that is, the same reference number indicates the same component throughout the specification.

In the drawings attached to this specification, the size, position, connection relationship, etc. of each component constituting the present invention may be described to be exaggerated, reduced, or omitted in order to convey the idea of the present invention sufficiently clearly or for convenience of explanation. Therefore, its proportions or scale may not be exact.

In addition, hereinafter, in describing the present invention, detailed descriptions of configurations that are judged to unnecessarily obscure the gist of the present invention, for example, known technologies including prior art, may be omitted.

Hereinafter, a method and device for monitoring brain water based on near-infrared spectroscopy according to a preferred embodiment of the present invention will be described in detail with reference to the attached drawings.

First, before explaining the method and device for monitoring brain water based on near-infrared spectroscopy according to the present invention, the near-infrared spectroscopy technique applied to the present invention will be described.

Near-infrared spectroscopy (NIRS) is a method that uses the near-infrared region of the electromagnetic spectrum (approximately 700 to 2,500 nm), which can be quickly determine the properties of materials by using the NIR reflectance spectrum to measure light scattered through a sample, without changing the sample.

Near-infrared spectrum penetrates the skull and reaches a depth of about 1 to 3 cm from the skull. At this time, the blood oxygen concentration and water change in the corresponding cortex can be measured based on the degree of scattering and reflection from the initial amount of near-infrared rays.

In other words, near-infrared spectroscopy can quantitatively evaluate tissue water, which is determined by blood, cerebrospinal fluid, interstitial fluid, and intracellular fluid, and indirectly measure neural activity in the area by measuring the degree of attenuation due to scattering and absorption influenced by changes in the concentration of oxidized and reduced hemoglobin due to neural activity.

Meanwhile, the glymphatic system mixes cerebrospinal fluid and interstitial fluid and removes waste products from the brain through solute exchange. Based on the fact that cerebrospinal fluid and interstitial fluid are composed of more than 99% water, it can be seen that changes in brain water during sleep is changes in the glymphatic system.

Accordingly, in the present invention, based on the fact that glymphatic system activation during sleep is accompanied by an increase in brain tissue water, the glymphatic system activity is quantitatively measured through brain water monitoring using near-infrared spectroscopy, and the measurement results may be used as brain health indicators and a key element in identifying the pathophysiology of various neurological diseases.

In addition, in the present invention, based on the fact that brain edema and increased intracranial pressure are accompanied by changes in brain tissue water, the near-infrared spectroscopy technique for measuring tissue water may be applied to acute monitoring of various brain diseases.

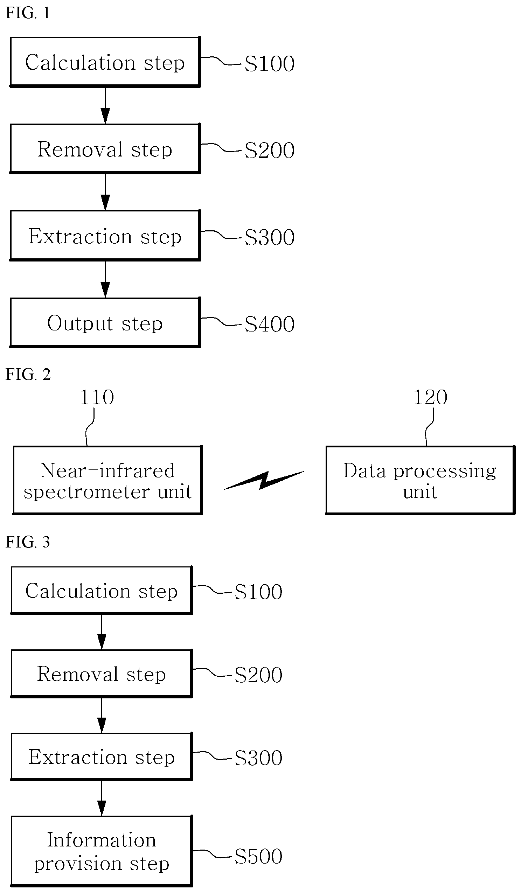

FIG. 1 is a processing diagram for explaining a method for monitoring brain water based on near-infrared spectroscopy according to an embodiment of the present invention.

As shown in FIG. 1, the method for monitoring brain water based on the near-infrared spectroscopy according to the present invention may comprise a calculation step (S100) of calculating oxidized hemoglobin, reduced hemoglobin, and water fraction, and a removal step (S200) of removing motion artifacts, an extraction step (S300) of extracting a pure water value, and an output step (S400) of outputting the extracted water value.

In the calculation step (S100), oxidized hemoglobin, reduced hemoglobin, and water fraction may be calculated based on signals measured using near-infrared spectroscopy.

In the calculation step (S100), the first wavelength (λ1), the second wavelength (λ2), and the third wavelength (λ3), which are wavelengths of different bands, may be used using the near-infrared spectroscopy technique.

In general, to increase MBLL (Modified Beer Lambert Law) accuracy, the first wavelength (λ1) may be selected from 650 to 800 nm, the second wavelength (λ2) may be selected from 800 to 900 nm, and the third wavelength (λ3) may be selected from 900 nm or more. Exemplarily, wavelengths in the 780nm, 850nm, and 925nm bands may be used as the first, second, and third wavelengths, respectively.

Oxidized hemoglobin, reduced hemoglobin, and water fraction may be calculated using the Modified Beer Lambert Law (MBLL) by applying several effective optical differential pathlength factor (DPF) and the unique extinction coefficient of each material for each wavelength value.

Here, the differential pathlength factor due to the first wavelength (λ1) is expressed as $Δ {OD}_{λ 1} = log \left(\frac{{I}_{λ 1} \left(t\right)}{{I}_{λ 1} \left(t=0\right)}\right)$, and the second wavelength (λ2) and third wavelength (λ3) may also be calculated in the same form.

The Modified Beer Lambert Law (MBLL) may be expressed as Formula 1 below. [Formula 1] $\left[\begin{matrix}Δ {C}_{HbO 2} \\ Δ {C}_{HbR} \\ Δ {F}_{water}\end{matrix}\right] = {\left[\begin{matrix}{ε}_{HbO , λ 1} & {ε}_{HbR , λ 2} & {ε}_{water , λ 3} \\ {ε}_{HbO 2 , λ 1} & {ε}_{HbR , λ 2} & {ε}_{water , λ 3} \\ {ε}_{HbO 2 , λ 1} & {ε}_{HbR , λ 2} & {ε}_{water , λ 3}\end{matrix}\right]}^{- 1} \left[\begin{matrix}\frac{Δ {OD}_{λ 1}}{{DPF}_{λ 1} \times L} \\ \frac{Δ {OD}_{λ 2}}{{DPF}_{λ 2} \times L} \\ \frac{Δ {OD}_{λ 3}}{{DPF}_{λ 3} \times L}\end{matrix}\right]$

In Formula 1, ε is the extinction coefficient, DPF is the differential path coefficient, L is the channel length (light source-detector distance), OD is the optical density, I is the luminous intensity, Δ*C*_{*HbO*2} is the oxidized hemoglobin concentration, Δ*C_{HbR}* is the reduced hemoglobin concentration, Δ*F_{water}* is the water fraction.

In the removal step (S200), motion artifacts may be removed.

Specifically, motion artifacts may be removed by applying a motion artifact removal algorithm to the oxidized hemoglobin concentration (Δ*C*_{*HbO*2}), reduced hemoglobin concentration (Δ*C_{HbR}*), and water fraction (Δ*F_{water}*) generated in the calculation step (S100).

After acquiring the measurement signal based on near-infrared spectroscopy, low-pass filtering may be performed as a preprocessing process to remove non-ideal contamination signals, i.e. motion artifacts, caused by heart rate, respiration, pulse, neural activity, and vascular movement.

Here, low-pass filtering may be performed using a low-pass filter (LPF) with a cutoff frequency of approximately 0.01 Hz.

Additionally, after detecting movement using a gyro sensor, motion artifacts of movement may be removed through signal restoration using spline interpolation in the post-processing process.

In one embodiment of the present invention, the removal step (S200) is implemented after performing the calculation step (S100), but in another embodiment, it may be implemented that the removal step (S200) is omitted and the extraction step (S300) is performed immediately after the calculation step (S100).

However, in order to accurately measure brain water activity, a step (extraction step) is necessary to extract the pure water value, rather than simply confirming the change in water absorption rate. This is the same in all methods for measuring the glymphatic system activity below.

In addition, in another embodiment, the removal step (S100) may be performed first, that is, the motion artifact of movement may be removed, and then the calculation step (S100) of calculating oxidized hemoglobin, reduced hemoglobin, and water fraction may be performed, and then the extraction step (S300) may be performed.

In the extraction step (S300), the signals of oxidized hemoglobin and reduced hemoglobin may be suppressed from the water signal in order to extract only pure water values.

At this time, linear minimum mean square error (LMMSE) may be applied to resolve cross-talk artifacts, in which signals interfere with each other and the measured value is completely canceled.

A decorrelation algorithm that subtracts the common portion of the oxidized hemoglobin signal and the reduced hemoglobin signal from the water value may be used in the extraction step (S300).

Ordinary least square regression may be used as a decorrelation algorithm.

Ordinary least squares regression (OLS regression) is a type of linear least squares method for estimating unknown parameters in a linear regression model.

Since the measured water (*Water_{measured}*) may be modeled using Formula 2 below, using ordinary least squares regression (OLS regression), the error may be reduced by minimizing the remaining terms except for *Water_{measured}*. [Formula 2] ${Water}_{measured} = {Water}_{target} + {β}_{1} Δ {C}_{HbO 2} + {β}_{2} Δ {C}_{HbR}$

As described above, by performing additional data processing on the signal measured in the calculation step (S100) through the removal step (S200) and the extraction step (S300), only the water component may be extracted, further more increasing the accuracy of the signal.

Thereafter, in the output step (S400), the extracted moisture value may be output.

In the output step (S400), information on the change in water value extracted through the calculation step (S100) to the extraction step (S300) above, that is, the change in brain water in a subject, may be output. Information about the change in brain water in the subject may be used in various forms.

Information about changes in brain water in the subject may be used to monitor the glymphatic system activity or to monitor severe brain diseases.

FIG. 2 is a diagram schematically showing the configuration of a device for monitoring brain water based on near-infrared spectroscopy according to an embodiment of the present invention.

As shown in Figure 2, the device for monitoring brain water based on near-infrared spectroscopy according to an embodiment of the present invention may comprise a near-infrared spectrometer unit (110) that outputs a near-infrared signal to a subject and receives a signal reflected from the subject; and a data processing unit (120) for processing the signal obtained from the near-infrared spectrometer unit (110).

The near-infrared spectroscopy unit (110) may comprise a light source (not shown) and a detector (not shown), and it is desirable to place the light source (not shown) and the detector (not shown) at a preset distance to allow incident photons to penetrate deep into brain tissue, for example, 3cm.

In an embodiment of the present invention, the near-infrared spectrometer unit (110) and the data processing unit (120) may be implemented to be connected based on a short-range wireless communication method, but are not limited to this.

A method for monitoring brain water based on signals acquired through the near-infrared spectroscopy unit (110) may be performed in the data processing unit (120) described above.

That is, in the data processing unit (120), the changes in brain water may be monitored by performing the calculation step (S100) to the output step (S400) described above.

In addition, the water value output from the data processing unit (120) may be used as a scale for evaluating the subject's glymphatic system and monitoring the subject's brain disease.

Here, brain disease may include degenerative brain disease, and may include changes in cerebral pressure accompanying cerebral edema or increased intracranial pressure due to stroke, brain lesion, head trauma, etc.

In one embodiment of the present invention, the glymphatic system activity man be measured using a method and device for monitoring brain water based on near-infrared spectroscopy.

FIG. 3 is a processing diagram for explaining a method for measuring the glymphatic system activity according to an embodiment of the present invention.

As shown in FIG. 3, the method for measuring the glymphatic system activity according to the present invention may comprise a calculation step (S100) of calculating oxidized hemoglobin, reduced hemoglobin, and water fraction, a removal step (S200) of removing motion artifacts, and an extraction step (S300) of extracting a pure water value, and an a information provision step (S500) of providing the glymphatic system activity information based on the water value extracted in the extraction step (S300).

Here, the operations of the calculation step (S100) to the extraction step (S300) are the same as the calculation step (S100) to the extraction step (S300) of the method for monitoring brain water based on the near-infrared spectroscopy according to an embodiment of the present invention, so the same reference numerals are given and repeated explanations are omitted.

In the information provision step (S500), the glymphatic system activity man be measured based on the water value extracted in the extraction step (S300), and information on the measured glymphatic system activity may be provided.

The information on the glymphatic system activity measured in the information provision step (S500) may appear similar to the brain water change output in the output step (S400) of the method for monitoring brain water based on the near-infrared spectroscopy according to an embodiment of the present invention.

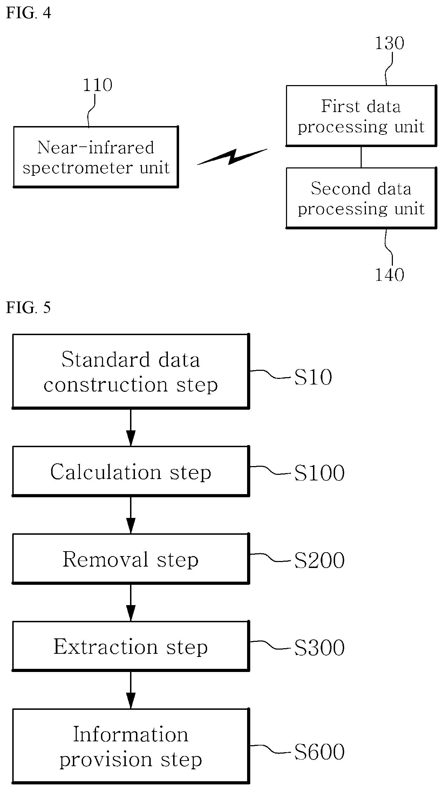

FIG. 4 is a diagram schematically showing the configuration of a device for measuring the glymphatic system activity according to an embodiment of the present invention.

As shown in FIG. 4, the device for measuring the glymphatic system activity according to the present invention may comprise a near-infrared spectrometer unit (110) that outputs a near-infrared signal to a subject and receives a signal reflected from the subject, a first data processing unit (130) for processing the signal obtained from the near-infrared spectrometer unit (110), and a second data processing unit (140) that provides information on the glymphatic system activity based on the water value output from the first data processing unit (130).

The second data processing unit (140) may measure the glymphatic system activity based on the water value output from the first data processing unit (130) and provide information on the measured glymphatic system activity.

The above-described first data processing unit (130) and second data processing unit (140) may measure the glymphatic system activity in conjunction with each other.

That is, the first data processing unit (130) performs the above-described calculation step (S100) to the extraction step (S300), and the second data processing unit (140) performs the above-described information provision step (S500) to measure the glymphatic system activity.

In an embodiment of the present invention, the first data processing unit (130) and the second data processing unit (140) are implemented to measure the glymphatic system activity in conjunction with each other, but it may be implemented to be performed in only one data processing unit (e.g., the first data processing unit).

In addition, in one embodiment of the present invention, the glymphatic system activity may be evaluated and related information may be provided by using the method and device for monitoring brain water based on near-infrared spectroscopy.

FIG. 5 is a processing diagram for explaining a method for evaluating the glymphatic system activity according to an embodiment of the present invention.

As shown in FIG. 5, the method for evaluating the glymphatic system activity according to the present invention may comprise a standard data construction step (S10) of constructing standard data of brain tissue water, and a calculation step (S100) for calculating oxidized hemoglobin, reduced hemoglobin, and water fraction, a removal step (S200) to remove motion artifacts, an extraction step (S300) to extract pure water values, and an information provision step (S600) of evaluating glymphatic system activity based on the water value extracted in the extraction step (S300) and the standard data of brain tissue water and providing evaluation information.

Here, the operations of the calculation step (S100) to the extraction step (S300) are the same as the calculation step (S100) to the extraction step (S300) of the method for monitoring brain water based on the near-infrared spectroscopy according to an embodiment of the present invention, so the same reference numerals are given and repeated explanations are omitted.

In the standard data construction stage (S10), standard data (representative values and distribution statistics) of brain tissue water may be constructed based on measurements taken in a representative population or condition-specific group selected in consideration of at least one of the following: age, gender, genotype (apolipoprotein E (ApoE) genotype, aquaporin-4 (AQP-4) genotype, and other related genotypes), brain structure (whole brain/white matter/cortical volume, volume and thickness of each cortex/white matter, fine brain structure, diffusion, brain network, etc.) and function (results of comprehensive or detailed cognitive function evaluation, motor concentration, daily living ability, etc.), comorbidities (high blood pressure, diabetes, hyperlipidemia, depression, etc.) and lifestyle (smoking, drinking, diet, exercise, etc.) related to glymphatic system activity.

Standard data on brain tissue water may be constructed according to wakefulness-sleep transition, each sleep stage, or accumulation of various sections (daily sleep time/NREM sleep/all REM sleep, all specific sleep stages, specific time sections, NREM sleep-REM sleep cycle, etc.).

Here, the standard data on brain tissue water according to wakefulness-sleep transition may be constructed based on the rate or extent of change in brain tissue water according to the transition between awakening-NREM sleep, NREM sleep-REM sleep, REM sleep-NREM sleep, NREM sleep-awakening, and REM sleep-awakening.

Standard data on brain tissue water for each stage of sleep may be constructed based on the absolute value of glymphatic system activity during NREM sleep (light sleep, deep sleep, or NREM sleep stages 1, 2, and 3, REM sleep) or the amount of change compared to awakening.

Meanwhile, in the information provision step (S600), the glymphatic system activity may be evaluated based on the water value extracted in the extraction step (S300) and the standard data of brain tissue water constructed in the step S10, and then evaluation information may be provided.

The evaluation of glymphatic system activity may be done by comparing the standard data of brain tissue water (representative values and distribution statistics) constructed in the step S10 above with the measurements of the evaluation target (individual/group disease, condition/characteristic).

In the step of comparing the measurement value of the evaluation target and the standard data of brain tissue water, if a significant difference occurs with the standard data (for example, if there is a difference of more than 2.0 - 3.0 standard deviations from the standard data value), categorization results corresponding to the difference (normal vs. abnormal (or strange); very good/good/average/bad/very bad, etc.) may be provided. In addition, the difference between standard data and the measurement value of the evaluation target may be provided by scoring it as a standardized value (absolute value, ratio, stratification section, etc.).

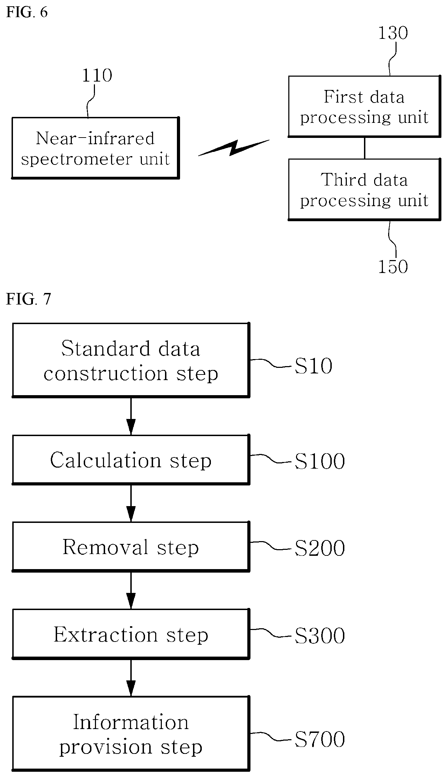

FIG. 6 is a diagram schematically showing the configuration of a device for evaluating the glymphatic system activity according to an embodiment of the present invention.

As shown in FIG. 6, the device for evaluating the glymphatic system activity according to the present invention may comprise a near-infrared spectrometer unit (110) that outputs a near-infrared signal to a subject and receives a signal reflected from the subject, a first data processing unit (130) for processing the signal obtained from the near-infrared spectrometer unit (110), and a third data processing unit (150) that provides evaluation information based on the water value output from the first data processing unit (130).

The third data processing unit (150) may measure the glymphatic system activity based on the water value output from the first data processing unit (130) and provide evaluation information on the glymphatic system activity.

The above-described first data processing unit (130) and third data processing unit (150) may measure the glymphatic system activity in conjunction with each other.

That is, the first data processing unit (130) performs the above-described calculation step (S100) to the extraction step (S300), and the third data processing unit (150) performs the above-described information provision step (S600) to measure the glymphatic system activity.

In order to evaluate the glymphatic system activity by performing the above-described information provision step (S600) in the third data processing unit (150), the third data processing unit (150) may store standard data on brain tissue water constructed according to wakefulness-sleep transition, each sleep stage, or accumulation of various sections, etc.

Additionally, the standard data on brain tissue water may be stored in a separate database (not shown).

In an embodiment of the present invention, the first data processing unit (130) and the third data processing unit (150) are implemented to measure the glymphatic system activity in conjunction with each other, but it may be implemented to be performed in only one data processing unit (e.g., the first data processing unit).

In addition, in one embodiment of the present invention, sleep states may be evaluated using the method and device for monitoring brain water based on near-infrared spectroscopy.

FIG. 7 is a processing diagram for explaining a method for evaluating sleep state according to an embodiment of the present invention.

As shown in FIG. 7, the method for evaluating sleep state according to the present invention may comprise a standard data construction step (S10) of constructing standard data of brain tissue water, a calculation step (S100) of calculating oxidized hemoglobin, reduced hemoglobin, and water fraction, a removal step (S200) of removing motion artifacts, and an extraction step (S300) of extracting a pure water value, and an information provision step (S700) of providing information on sleep state according to the glymphatic system activity for wakefulness-sleep or sleep stage based on the water value extracted in the extraction step (S300) and standard data on brain tissue water.

Here, the operations of the calculation step (S100) to the extraction step (S300) are the same as the calculation step (S100) to the extraction step (S300) of the method for monitoring brain water based on the near-infrared spectroscopy according to an embodiment of the present invention, so the same reference numerals are used. is given and repeated explanations are omitted.

In addition, the operation of the standard data construction stage (S10) is the same as the standard data construction stage (S10) of the method for evaluating the glymphatic system activity according to an embodiment of the present invention, so the same reference numerals are used. is given and repeated explanations are omitted.

In the information provision step (S700), based on the water value extracted in the extraction step (S300) and the standard data on brain tissue water constructed in the step S10, information on sleep state according to the glymphatic system activity during sleep for the awakening-sleep or sleep stage (AWAKE->NREM, NREM->AWAKE, REM->NREM, NREM->REM) may be provided.

Specifically, in the information provision step (S700), the change in water fraction according to the sleep stage transition may be monitored and compared with the standard data on brain tissue water constructed in the step S10, and based on the comparison results, information about sleep state according to the change in the glymphatic system activity during sleep, that is, the appropriateness of the sleep state according to the glymphatic system activity may be provided.

When providing information on sleep state, the measurement value of the monitored evaluation target may be compared with the standard data of brain tissue water, and the difference may be provided by scoring it as a standardized value (absolute value, ratio, stratification section, etc.). Additionally, if a significant difference occurs with the measurement value of the monitored evaluation target and the standard data of brain tissue water, categorization results corresponding to the difference (normal vs. abnormal (or strange); very good/good/average/bad/very bad, etc.) may be provided.

Meanwhile, in the information provision step (S700), based on the water value extracted in the extraction step (S300), the change in water fraction according to the sleep stage transition from awakening to sleep may be monitored to provide information on sleep state according to the change in glymphatic system activity.

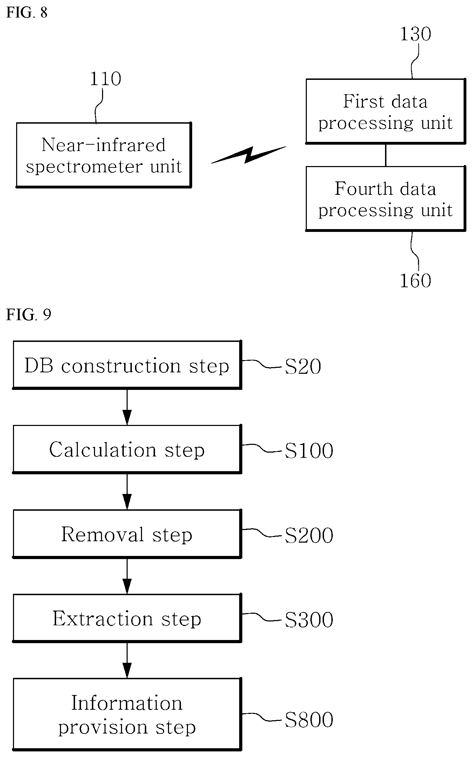

FIG. 8 is a diagram schematically showing the configuration of a device for evaluating sleep state according to an embodiment of the present invention.

As shown in FIG. 8, the device for evaluating sleep state according to the present invention may comprise a near-infrared spectrometer unit (110) that outputs a near-infrared signal to a subject and receives a signal reflected from the subject, a first data processing unit (130) for processing the signal obtained from the near-infrared spectrometer unit (110), and a fourth data processing unit (160) that provides information on sleep state according to the change in the glymphatic system activity for the awakening-sleep or sleep stage based on the water value output from the first data processing unit (130).

The fourth data processing unit (160) may monitor the change in water fraction according to the sleep stage transition based on the water value output from the first data processing unit (130) and provide information on sleep state for the awakening-sleep or sleep stage.

The above-described first data processing unit (130) and fourth data processing unit (160) may measure the glymphatic system activity in conjunction with each other.

That is, the first data processing unit (130) performs the above-described calculation step (S100) to the extraction step (S300), and the fourth data processing unit (160) performs the above-described information provision step (S700) to provide information on sleep state for the awakening-sleep or sleep stage.

In order to provide information on sleep state for the awakening-sleep or sleep stage by performing the above-described information provision step (S700) in the fourth data processing unit (160), the fourth data processing unit (160) may store standard data on brain tissue water constructed according to wakefulness-sleep transition, each sleep stage, or accumulation of various sections, etc.

Additionally, the standard data on brain tissue water may be stored in a separate database (not shown).

In an embodiment of the present invention, the first data processing unit (130) and the fourth data processing unit (160) are implemented to measure the glymphatic system activity in conjunction with each other, but it may be implemented to be performed in only one data processing unit (e.g., the first data processing unit).

In addition, in one embodiment of the present invention, auxiliary information related to brain disease, specifically neurodegenerative brain disease, may be provided using the method and device for monitoring brain water based on near-infrared spectroscopy.

Specifically, it may provide neurodegenerative brain disease indicators corresponding to the glymphatic system activity, measures the glymphatic system activity based on the change in brain water measured based on near-infrared spectroscopy, and provides auxiliary information related to prediction, progression, diagnosis, and treatment of neurodegenerative brain disease based on an indicator corresponding to the measured glymphatic system activity.

FIG. 9 is a processing diagram for explaining a method of providing auxiliary information related to neurodegenerative brain disease according to an embodiment of the present invention.

As shown in FIG. 9, the method of providing auxiliary information related to neurodegenerative brain disease according to the present invention may comprises a DB construction step (S20) of constructing a database comprising neurodegenerative brain disease indicators corresponding to the glymphatic system activity; a calculation step (S100) of calculating oxidized hemoglobin, reduced hemoglobin, and water fraction, a removal step (S200) of removing motion artifacts, and an extraction step (S300) of extracting a pure water value, and an information provision step (S800) of providing auxiliary information related to prediction, progression, diagnosis, and treatment of neurodegenerative brain disease based on the water value extracted in the extraction step (S300) and the database.

Here, the operations of the calculation step (S100) to the extraction step (S300) are the same as the calculation step (S100) to the extraction step (S300) of the method for monitoring brain water based on the near-infrared spectroscopy according to an embodiment of the present invention, so the same reference numerals are used. is given and repeated explanations are omitted.

The DB construction step (S20) may be implemented similarly to the standard data construction step (S10) described above. That is, a database comprising neurodegenerative brain disease indicators corresponding to the glymphatic system activity may be constructed based on measurements taken in a representative population or condition-specific group selected in consideration of at least one of the following: age, gender, genotype (apolipoprotein E (ApoE) genotype, aquaporin-4 (AQP-4) genotype, and other related genotypes), brain structure (whole brain/white matter/cortical volume, volume and thickness of each cortex/white matter, fine brain structure, diffusion, brain network, etc.) and function (results of comprehensive or detailed cognitive function evaluation, motor concentration, daily living ability, etc.), comorbidities (high blood pressure, diabetes, hyperlipidemia, depression, etc.) and lifestyle (smoking, drinking, diet, exercise, etc.) related to glymphatic system activity.

When constructing a database comprising neurodegenerative brain disease indicators in the DB construction stage (S20), standard data on brain tissue water may also be constructed.

In the information provision step (S800), auxiliary information related to prediction, progression, diagnosis, and treatment of neurodegenerative brain diseases may be provided based on the water value extracted in the extraction step (S300) and the database constructed in the DB construction step (S20).

Specifically, in the information provision step (S800), the glymphatic system activity may be measures based on the water fraction extracted in the extraction step (S300), and a neurodegenerative brain disease indicator corresponding to the measured glymphatic system activity may be searched in the database, and then auxiliary information related to prediction, progression, diagnosis, and treatment of neurodegenerative brain disease may be provided based on the searched indicator.

When providing auxiliary information in the information provision step (S800), the measured value of the monitored evaluation target and the standard data on brain tissue water may be compared, and the difference may be provided by scoring it with a standardized value (absolute value, ratio, stratification section, etc.). Additionally, if a significant difference occurs with the measurement value of the monitored evaluation target and the standard data of brain tissue water, categorization results corresponding to the difference may be provided.

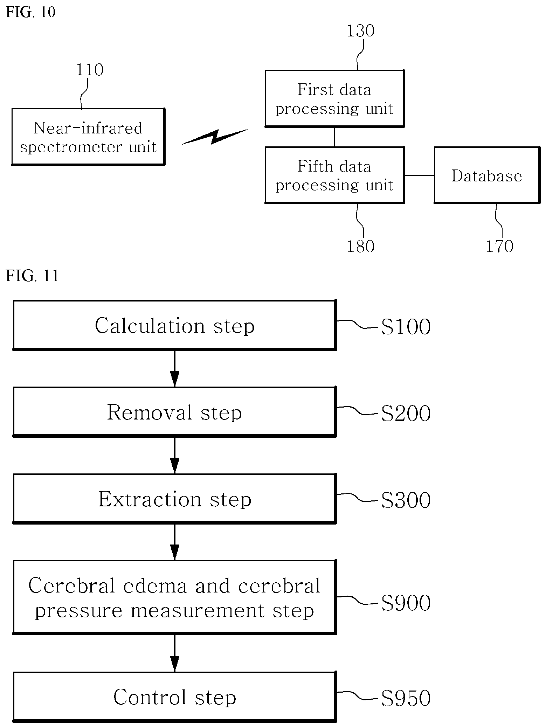

FIG. 10 is a diagram schematically showing the configuration of a device for providing auxiliary information related to neurodegenerative brain disease according to an embodiment of the present invention.

As shown in FIG. 10, the device for providing auxiliary information related to neurodegenerative brain disease according to the present invention may comprise a near-infrared spectrometer unit (110) that outputs a near-infrared signal to a subject and receives a signal reflected from the subject, a first data processing unit (130) for processing the signal obtained from the near-infrared spectrometer unit (110), database (170) comprising neurodegenerative brain disease indicators corresponding to glymphatic system activity, and a fifth data processing unit (180) that provides auxiliary information related to prediction, progression, diagnosis, and treatment of neurodegenerative brain disease based on the water value output from the first data processing unit (130) and the database (170).

The fifth data processing unit (180) may measure the glymphatic system activity based on the water fraction output from the first data processing unit (130), and a neurodegenerative brain disease indicator corresponding to the measured glymphatic system activity may be searched in the database (170), and then auxiliary information related to prediction, progression, diagnosis, and treatment of neurodegenerative brain disease may be provided based on the searched indicator.

The above-described first data processing unit (130) and fifth data processing unit (180) may measure the glymphatic system activity in conjunction with each other.

That is, the first data processing unit (130) performs the above-described calculation step (S100) to the extraction step (S300), and the fifth data processing unit (180) performs the above-described information provision step (S800) to provide auxiliary information related to neurodegenerative brain disease.

In an embodiment of the present invention, the first data processing unit (130) and the fifth data processing unit (180) are implemented to provide auxiliary information related to neurodegenerative brain disease in conjunction with each other, but it may be implemented to be performed in only one data processing unit (e.g., the first data processing unit).

In addition, in one embodiment of the present invention, cerebral edema and cerebral pressure may be measured and related information may be provided by using the method and device for monitoring brain water based on near-infrared spectroscopy.

FIG. 11 is a processing diagram for explaining a method of measuring cerebral edema and cerebral pressure according to an embodiment of the present invention.

As shown in FIG. 11, the method for measuring cerebral pressure according to the present invention may comprises a calculation step (S100) of calculating oxidized hemoglobin, reduced hemoglobin, and water fraction, a removal step (S200) of removing motion artifacts, and an extraction step (S300) of extracting a pure water value, and a cerebral edema and cerebral pressure measurement step (S900) of measuring the cerebral edema and cerebral pressure based on the water value extracted in the extraction step (S300). Furthermore, it may further comprise a control step (S950) of controlling the cerebral pressure regulator based on the measured cerebral pressure in the cerebral edema and cerebral pressure measurement step (S900).

Here, the operations of the calculation step (S100) to the extraction step (S300) are the same as the calculation step (S100) to the extraction step (S300) of the method for monitoring brain water based on the near-infrared spectroscopy according to an embodiment of the present invention, so the same reference numerals are used. is given and repeated explanations are omitted.

In the cerebral edema and cerebral pressure measurement step (S900), the cerebral edema and cerebral pressure may be measured based on the water value extracted in the extraction step (S300), and the measured cerebral edema and cerebral pressure information may be provided.

When providing information on cerebral edema and cerebral pressure, the amount of brain tissue water may be measured every certain cycle, taking into account the diurnal change (daytime, night) of the evaluation target and the range of change according to conditions (blood pressure, respiration, arterial blood oxygen partial pressure, body water amount index, etc.) By comparing this to the initial value, for the difference in the measured value at a certain period, categorization results corresponding to the difference (normal vs. abnormal; stratification section - e.g., mild, moderate, severe, etc.) or a standardized value (absolute value, ratio, cumulative change, etc.) may be provided by scoring it to provide information on cerebral edema and cerebral pressure.

In the control step (S950), a cerebral pressure regulator such as a shunt may be controlled based on the cerebral pressure information measured in the cerebral edema and intracranial pressure measurement step (S900).

Specifically, if the cerebral pressure information measured in the cerebral edema and cerebral pressure measurement step (S900) exceeds a preset range, the cerebrospinal fluid discharge passage of the shunt may be controlled to be opened.

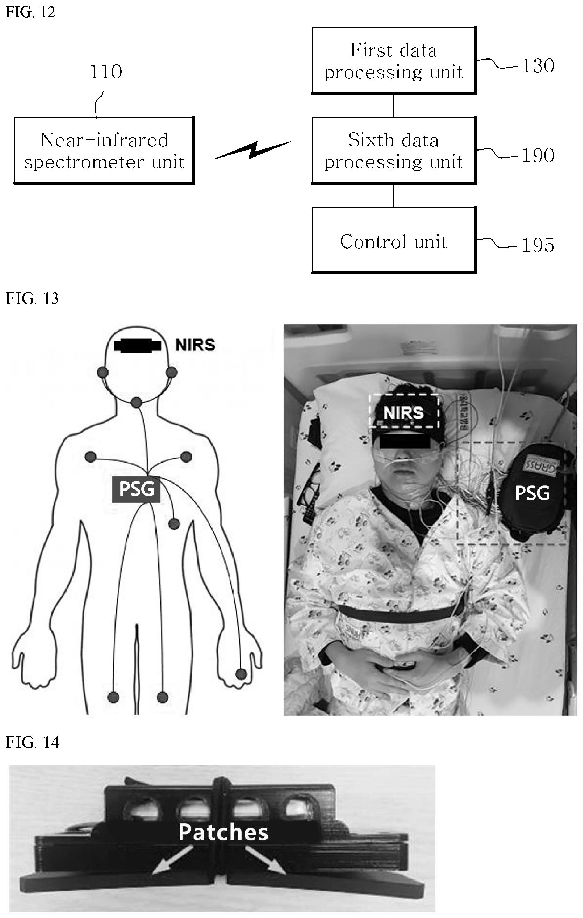

FIG. 12 is a diagram schematically showing the configuration of a device for measuring cerebral pressure according to an embodiment of the present invention.

As shown in FIG. 12, the device of measuring cerebral pressure according to the present invention may comprise a near-infrared spectrometer unit (110) that outputs a near-infrared signal to a subject and receives a signal reflected from the subject, a first data processing unit (130) for processing the signal obtained from the near-infrared spectrometer unit (110), and a sixth data processing unit (190) that provides information on cerebral edema and cerebral pressure based on the water value output from the first data processing unit (130). Furthermore, it may further comprise a control unit (195) that controls the cerebral pressure regulator based on the cerebral pressure output from the sixth data processing unit (190).

The sixth data processing unit (190) may measure cerebral edema and cerebral pressure based on the water value output from the first data processing unit (130) and provide information on the measured cerebral edema and cerebral pressure.

The control unit (195) may control the cerebral pressure regulator such as shunts based on the cerebral pressure information output from the sixth data processing unit (190).

Specifically, if the cerebral pressure information output from the sixth data processing unit (190) exceeds a preset range, the cerebrospinal fluid discharge passage of the shunt may be controlled to be opened.

The above-described first data processing unit (130) and sixth data processing unit (190) may provide the cerebral pressure information in conjunction with each other.

That is, the first data processing unit (130) performs the above-described calculation step (S100) to the extraction step (S300), and the sixth data processing unit (190) performs the above-described cerebral pressure measurement step (S900) to provide the cerebral pressure information.

In an embodiment of the present invention, the first data processing unit (130) and the sixth data processing unit (190) are implemented to provide the cerebral pressure information in conjunction with each other, but it may be implemented to be performed in only one data processing unit (e.g., the first data processing unit).

The method according to the embodiments described above and the operation by the device for performing the method may be at least partially implemented as a computer program and stored in a computer-readable recording medium.

For example, it may be implemented with a program product comprised of a computer-readable medium containing program code, which can be executed by a processor to perform any or all steps, operations, or processes described.

The computer may be any device that may be integrated with or be a computing device, such as a desktop computer, laptop computer, notebook, smart phone, or the like. A computer is a device that has one or more alternative, special-purpose processors, memory, storage, and networking components (either wireless or wired). For example, the computer may run an operating system such as Microsoft's Windows-compatible operating system, Apple's OS X or iOS, a Linux distribution, or Google's Android OS.

The computer-readable recording medium includes all types of recording and identification devices that store data that can be read by a computer. Examples of computer-readable recording media include ROM, RAM, CD-ROM, magnetic tape, floppy disk, and optical data storage identification devices. Additionally, computer-readable recording media may be distributed across computer systems connected to a network, and computer-readable codes may be stored and executed in a distributed manner. Additionally, functional programs, codes, and code segments for implementing this embodiment can be easily understood by those skilled in the art to which this embodiment belongs.

Embodiments of the invention are summarized in the following list of embodiments:
1. A method for monitoring brain water based on near-infrared spectroscopy, performed by a processor, which is characterized by comprising:
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an output step of outputting the water fraction extracted in the extraction step.
2. The method according to embodiment 1,
   wherein the method is characterized by further comprising a removal step of removing motion artifacts from the signal measured using the near-infrared spectroscopy,
   wherein the removal step of is performed before or after the calculation step.
3. The method according to embodiment 2,
   wherein the removal step is characterized by removing motion artifacts by low-pass filtering the signal measured using the near-infrared spectroscopy and using spline interpolation.
4. The method according to embodiment 1,
   wherein the extraction step is characterized by extracting a pure water fraction by removing the oxidized hemoglobin fraction and the reduced hemoglobin fraction from the water fraction calculated in the calculation step.
5. The method according to embodiment 1,
   wherein the extraction step is characterized by removing the oxidized hemoglobin fraction and reduced hemoglobin fraction from the water fraction calculated in the calculation step, but extracting a pure water fraction by removing the oxidized hemoglobin fraction and reduced hemoglobin fraction from the water fraction calculated using Linear Minimum Mean Square Error (LMMSE).
6. A method for measuring glymphatic system activity, performed by a processor, which is characterized by comprising:
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an information provision step of providing information on glymphatic system activity based on the water fraction extracted in the extraction step.
7. The method according to embodiment 6,
   wherein the information provision step is characterized by measuring glymphatic system activity based on the water fraction in the extraction step and providing information on the measured the glymphatic system activity.
8. A method for evaluating glymphatic system activity, performed by a processor, which is characterized by comprising:
   a standard data construction step of constructing standard data of brain tissue water based on measurements taken in the selected group;
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an information provision step of providing information comprising evaluating glymphatic system activity based on the water fraction extracted in the extraction step and the standard data of brain tissue water and providing evaluation information.
9. The method according to embodiment 8,
   wherein the information provision step is characterized by comparing the water fraction extracted in the extraction step with the standard data of brain tissue water, and if a significant difference occurs with the standard data of brain tissue water, providing information by scoring it with a categorization result or standardized value corresponding to the difference.
10. A method for evaluating sleep state, performed by a processor, which is characterized by comprising:
   a standard data of brain tissue water based on measurements taken in the selected group;
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an information provision step of providing information on the sleep state according to glymphatic system activity in wakefulness-sleep or sleep stages based on the water fraction extracted in the extraction step and the standard data of brain tissue water.
11. The method according to embodiment 10,
   wherein the information provision step is characterized by comparing the water fraction extracted in the extraction step with the standard data of brain tissue water, and if a significant difference occurs with the standard data of brain tissue water, providing information on the sleep state according to glymphatic system activity by scoring it with a categorization result or standardized value corresponding to the difference.
12. The method according to embodiment 10,
   wherein the information provision step is characterized by providing information on the sleep state according to glymphatic system activity by monitoring changes in the water fraction according to sleep stage transition based on the water fraction extracted in the extraction step.
13. A method for providing auxiliary information related to neurodegenerative brain disease, performed by a processor, which is characterized by comprising:
   a database (DB) construction step of constructing database comprising neurodegenerative brain disease indicators corresponding to glymphatic system activity;
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an information provision step of providing auxiliary information related to neurodegenerative brain disease based on the water fraction extracted in the extraction step and the database.
14. The method according to embodiment 13,
   wherein the information provision step is characterized by measuring glymphatic system activity based on the water fraction extracted in the extraction step, searching a neurodegenerative brain disease indicator corresponding to the measured the glymphatic system activity in the database, and then providing auxiliary information related to prediction, progression, diagnosis, and treatment of neurodegenerative brain disease based on the searched indicator.
15. The method according to embodiment 13,
   wherein the DB construction step constructs standard data of brain tissue water, and
   wherein the information provision step is characterized by comparing the water fraction extracted in the extraction step with the standard data of brain tissue water, and if a significant difference occurs with the standard data of brain tissue water, providing auxiliary information related to prediction, progression, diagnosis, and treatment of neurodegenerative brain disease by scoring it with a categorization result or standardized value corresponding to the difference.
16. A method for measuring cerebral edema and cerebral pressure, performed by a processor, which is characterized by comprising:
   a calculation step of calculating oxidized hemoglobin, reduced hemoglobin, and water fraction based on signals measured by near-infrared spectroscopy;
   an extraction step of extracting a pure water fraction from the water fraction calculated in the calculation step; and
   an information provision step of providing information comprising measuring cerebral edema and cerebral pressure based on the water fraction extracted in the extraction step and providing information on the measured cerebral edema and cerebral pressure.
17. The method according to embodiment 16,
   wherein the information provision step is characterized by measuring water amount of brain tissue at regular intervals, comparing it with the initial value, and providing information on the cerebral edema and cerebral pressure by scoring the difference with a categorization result or standardized value corresponding to the difference.
18. The method according to embodiment 16,
   wherein the method is characterized by further comprising:
   a control step of controlling the cerebral pressure regulator based on the cerebral pressure information measured in the providing information.
19. A device for monitoring brain water based on near-infrared spectroscopy, comprising:
   a near-infrared spectrometer unit that outputs a near-infrared signal to a subject and receives a signal reflected from the subject; and
   a data processing unit for processing the signal obtained from the near-infrared spectrometer unit,
   wherein the data processing unit is characterized in monitoring brain water changes by performing the near-infrared spectroscopy-based brain water monitoring method according to any one of embodiments 1 to 5.
20. A device for measuring glymphatic system activity, comprising:
   a near-infrared spectrometer unit that outputs a near-infrared signal to a subject and receives a signal reflected from the subject; and
   a data processing unit for processing the signal obtained from the near-infrared spectrometer unit,
   wherein the data processing unit is characterized by measuring glymphatic system activity by performing the method for measuring glymphatic system activity according to embodiment 6 or 7.
21. A device for evaluating glymphatic system activity, comprising:
   a near-infrared spectrometer unit that outputs a near-infrared signal to a subject and receives a signal reflected from the subject; and
   a data processing unit for processing the signal obtained from the near-infrared spectrometer unit,
   wherein the data processing unit is characterized by comprising standard data of brain tissue water and evaluating glymphatic system activity by performing the method for evaluating glymphatic system activity according to embodiment 8 or 9.
22. A device for evaluating a sleep state, comprising:
   a near-infrared spectrometer unit that outputs a near-infrared signal to a subject and receives a signal reflected from the subject; and
   a data processing unit for processing the signal obtained from the near-infrared spectrometer unit,
   wherein the data processing unit is characterized by providing information on the sleep state according to glymphatic system activity in wakefulness-sleep or sleep stages by performing the method for evaluating a sleep state according to any one of embodiments 10 to 12.
23. A device for providing auxiliary information related to neurodegenerative brain disease, comprising:
   a near-infrared spectrometer unit that outputs a near-infrared signal to a subject and receives a signal reflected from the subject;
   a data processing unit for processing the signal obtained from the near-infrared spectrometer unit; and
   database comprising neurodegenerative brain disease indicators corresponding to glymphatic system activity,
   wherein the data processing unit is characterized by providing auxiliary information related to neurodegenerative brain disease by performing the method for providing auxiliary information related to neurodegenerative brain disease according to any one of embodiments 13 to 15.
24. A device for measuring cerebral edema and cerebral pressure, comprising:
   a near-infrared spectrometer unit that outputs a near-infrared signal to a subject and receives a signal reflected from the subject; and
   a data processing unit for processing the signal obtained from the near-infrared spectrometer unit,
   wherein the data processing unit is characterized by providing information on the measured cerebral edema and cerebral pressure by performing the method for measuring cerebral edema and cerebral pressure according to any one of embodiments 16 to 18.
25. The device for measuring cerebral edema and cerebral pressure according to embodiment 23,
   wherein the device is characterized by further comprising a control unit to control the cerebral pressure regulator based on the cerebral pressure output from the data processing unit.

### [Experimental Example 1]

In one embodiment, the optimal algorithm for measuring the glymphatic system was applied to measure glymphatic system activity according to wakefulness-sleep and sleep stages (NREM sleep-REM sleep) and NREM sleeps (compared to NREM sleep at the beginning of sleep and NREM sleep at the end of sleep). Consistent with the results confirmed in animal experiments, dynamic changes were confirmed that glymphatic system activity is activated during sleep and suppressed upon awakening, and depending on the sleep stage, is activated in NREM sleep (also, the early NREM sleep is more active than the last NREM sleep) and suppressed in REM sleep.

Experimental examples according to this is as follows.

### Experimental Procedure

As shown in FIG. 13, 63 participants wore both polysomnography (PSG) and NIRS probes, and then slept around 21:00 to 22:00. The participants maintained their usual sleeping habits of going to bed (22:00-00:00) and waking up (07:00-09:00), which were generally consistent with the self-reported sleep time and sleeping at least 7 hours per night. Measurements started 30 minutes before falling asleep and continued for 60 minutes after waking up to compare changes in brain water upon waking up with changes in brain water in other states.

FIG. 14 is a diagram showing an exemplary NIRS device applied to this experiment. The NIRS device consists of a control board and two optode patches that simultaneously measure the left and right prefrontal cortex.

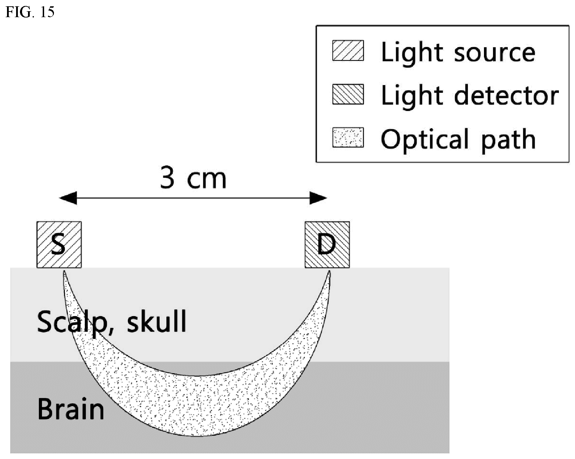

FIG. 15 is a diagram showing an exemplary NIRS measurement optical path, in which light is irradiated from a light source (S) and light reflected from a detector (D) located a certain distance away is measured.

### Polysomnography sleep recording

The polysomnographic (PSG) device used in this study was the Grass^{®} Comet-PLUS^{®}EEG/PSG equipment (Natus Medical Incorporated, San Carlos, CA, USA) and used standardized electrodes and detectors. Brain wave electrodes were attached to F3-A2, F4-A1, C3-A2, C4-A1, O1-A2, and O2-A1, and sampled at 200Hz using a 1.0Hz - 70Hz bandpass filter. Two safety sensors (eletrooculography, EOG) were attached to the outside of both eyes, a submentalis muscle conduction sensor was attached onto the submentalis muscle, and an electromyography sensor was also attached to both tibialis anterior muscles to record movements of the lower limbs during sleep.

Chest and abdominal imaging belts, airflow, oxygen saturation, snoring, body position, and electrocardiogram were also recorded.

Nocturnal sleep stages for every 30-second epoch, including wakefulness, N1, N2, N3, and REM sleep, were scored according to American Academy of Sleep Medicine (AASM) standard criteria by a well-trained sleep technologist. Apnea was defined as a decrease in airflow by more than 90% from baseline for more than 10 seconds despite sustained respiratory effort, and hypopnea was defined as a decrease in airflow signal by more than 30% from baseline for more than 10 seconds with oxygen desaturation of more than 4%. The scale used to assess the presence of OSA is the apnea-hypopnea index (AHI) or oxygen desaturation index (ODI), with AHI ≥10 or ODI ≥5 classifying patients as having OSA.

Limb movements were scored according to standard AASM criteria in which individual limb movements were recorded if their duration was between 0.5 and 10 seconds and the apparent EMG amplitude was 8 µV or greater than the resting EMG. Periodic limb movements (PLM) are defined as four or more consecutive limb movements with individual movements separated by intervals of 5 to 90 seconds. PLM index (PLMI) is the total number of PLMs per hour of sleep, and patients with PLMI of 15 or higher were considered to have periodic limb movements during sleep (PLMS). Total sleep time (TST), sleep efficiency (SE), sleep onset latency (SOL), wake time after sleep onset (WASO), NREM and REM stages, and AHI were measured.

### NIRS protocol

We continuously monitor the water content of the brain cortex during sleep using a custom-designed wireless NIRS device (FIG. 14). The NIRS device is designed to wirelessly transmit collected data to a receiver located nearby for a certain period of time (e.g., 10 hours) or more. The laser and detector of the NIRS device are spaced a predetermined distance (e.g., 3 cm) apart to allow incident light to penetrate deep into brain tissue.

In this experiment, three wavelengths are selected: 780nm, 850nm, and 925nm. The wavelength of 925nm plays an important role in estimating water content due to its high sensitivity to water.

Changes in water, oxy hemoglobin (HbO2), and deoxy hemoglobin (HbR) were tracked using the Modified Beer Lambert Law (MBLL), where the extinction coefficient and differential partial length factors (DPF) at each wavelength were retrieved from previous studies.

A low-pass filter with a cutoff frequency of 0.01 Hz was used to remove non-ideal contaminating signals caused by heart rate, respiration, neural activity, and vascular movement. A gyro sensor was used to sense (detect) movements, and motion artifacts were removed using spline interpolation in the post-processing step. Since the extracted water contains crosstalk from the hemoglobin signal and undesirable plasma fluid, we used a static linear least mean square estimator (LMMSE) to minimize the correlation between the water signal and the hemoglobin signal to exclude crosstalk and plasma fluid.

Water content extracted from the left and right hemispheres of the brain is averaged to reduce postural effects.

### Analysis of transitions between different types of sleep stages

Changes in water content were monitored during sleep stage transitions between one source and one destination stage to investigate the relationship between brain activity and water content.

### Transition analysis was performed for four sleep stage transitions (AWAKE→NREM, NREM→AWAKE, REM→NREM, and NREM→REM).

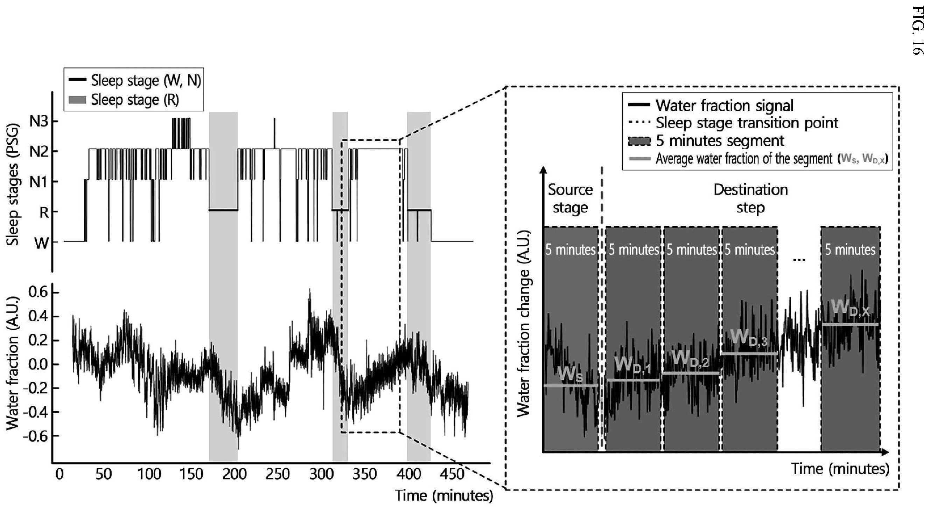

Time series data during sleep stage transitions were normalized to include 5 minutes of data from the source stage, as shown in FIG. 16. The destination stage was maintained until there was no interruption from other sleep stages for more than 2 minutes. The extracted water fraction data was divided into time lengths of 5 minutes and block averaged (W_{S}, W_{D,1}, W_{D,2}, ..., W_{D,X})).

A T-test was performed with the difference in water between each segment in the destination stage and a segment in the source stage (ΔH₂O(X) = W_{D,X} - W_{S}). Since the sleep time in each stage varies depending on individual differences, the number of subjects in each segment is different. All segments containing less than 10 subjects are also excluded. If a single subject has multiple sleep stage transitions, the water content of each sleep stage is averaged and used in the analysis.

### Comparison of water changes between the first and last NREM stages

To compare glymphatic system activity in total sleep, transition analysis was performed independently for the first NREM and last NREM and the results were compared.

The last NREM stage generally followed the REM stage, compared to the first NREM stage following the AWAKE stage. Due to the inconsistency in the source stage, the water fraction change during the first 5-minute interval (W_{D,1}) of the destination stage was used as a baseline to determine water change instead of using the source stage interval (W_{S}).

A paired t-test was performed between water content changes (ΔH₂O(X) = W_{D,X} - W_{D,1}) for each X segment of the destination stage, and analysis was performed independently for the first NREM and last NREM.

### Comparison of glymphatic system activity during REM stage

To investigate the relationship between glymphatic system activity and the REM cycle, we compared water content changes during the REM stage. If there were four or more of NREM -> REM transitions in the measurement, the NREM -> REM transition with REM >10 minutes was used for analysis.

We then performed a repeated measures ANOVA on changes in the water content between the source NREM stage segment and segment where the greatest difference was found in the transition analysis for each REM stage (ΔH₂O = W_{D,X} - W_{S}).

All statistical analyzes were performed using SPSS software version 22 (IBM, Armonk, NY) and MATLAB.

In FIG. 16, on the left is an exemplary diagram showing typical NIRS transient measurement results of water fraction in various sleep stages, and on the right is an exemplary diagram showing water fraction in sleep stage transitions between source and destination stages, The water fraction increases in the NREM stage but decreases in the AWAKE and REM stages. The dotted line in FIG. 16 captures an example event of a sleep stage transition. The water signal was split into time lengths of 5 minutes and block averaged. W_{S} represents the average water content of the source stage, and W_{D,X} represents the average water content of the Xth section of the destination stage.

### RESULT

### Research population

A total of 65 potential participants participated in the study, but among the 65 participants, 3 participants were excluded from the study due to high PSQI scores. A total of 62 participants participated in the Glymphatic-sleep experiment using NIRS and PSG. Additionally, 21 participants were excluded due to low signal-to-noise ratio (SNR) or sleep disorders such as OSA (AHI ≥10 or ODI ≥5) and PLMS (PLMI ≥15).

Therefore, the final study population was 41 participants (male = 24, mean age = 26.9 ± 7.7; see Table 1 for demographic and sleep characteristics in addition to PSG measurements). The mean TST was 6.4 hours (range, 3.2 - 7.7 hours) and the mean SE was 79.4% (range, 43.0% - 96.7%). The average percentage of total sleep time was 17.3% in N1 sleep, 60.3% in N2 sleep, 2.4% in N3 sleep, and 20.1% in REM sleep. The average minimum oxygen saturation was 92.2%, ranging from 83.0% to 96.0%. Although TST was shorter than the recommended sleep time of 7 hours per night, it was close to the TST of the general population in Korea. The low SE and N3 percentages observed in this experiment can be attributed to the first night effect and racial differences in slow wave sleep (SWS), which are less than 5% in the general Korean population.

**[Table 1]**

| **Variables** | **All participants (n** = **41)** |
|---|---|
| Demographics and sleep characteristics | |
| Age (years) | 28.9 ± 7.7 |
| Male, n (%) | 24 (58.5) |
| Body mass index (cm/m²) | 22.0 ± 2.3 |
| Insomnia severity index | 3.6 ± 3.6 |
| Morningness-eveningness questionnaire | 50.6 ± 8.1 |
| Epworth sleepiness scale | 5.5 ± 3.5 |
| Pittsburgh sleep quality index | 3.6 ± 1.6 |

| Polysomnography measures | |
|---|---|
| Total sleep time (hour) | 6.4 ± 1.1 |
| Sleep efficiency (%) | 79.4 ± 12.3 |
| Sleep onset latency (minutes) | 19.8 ± 24.0 |
| Wake after sleep onset (minutes) | 74.4 ± 50.1 |
| N1 (%) | 17.3 ± 8.1 |
| N2 (%) | 60.3 ± 7.8 |
| N3 (%) | 2.4 ± 3.5 |
| REM (%) | 20.1 ± 6.7 |
| N1 (minutes) | 63.1 ± 25.1 |
| N2 (minutes) | 232.1 ± 53.1 |
| N3 (minutes) | 9.0 ± 13.6 |
| REM (minutes) | 78.0 ± 33.8 |
| Apnea-hypopnea index (events/h) | 1.2 ± 1.7 |
| Oxygen desaturation index (events/h) | 1.2 ± 1.3 |
| Arousal index (events/h) | 21.6 ± 10.2 |
| Minimum oxygen saturation in sleep | 92.2 ± 2.7 |

| | |
|---|---|
| Data presented in forms of mean ± standard deviation or number in percentage. | |

### Transition analysis

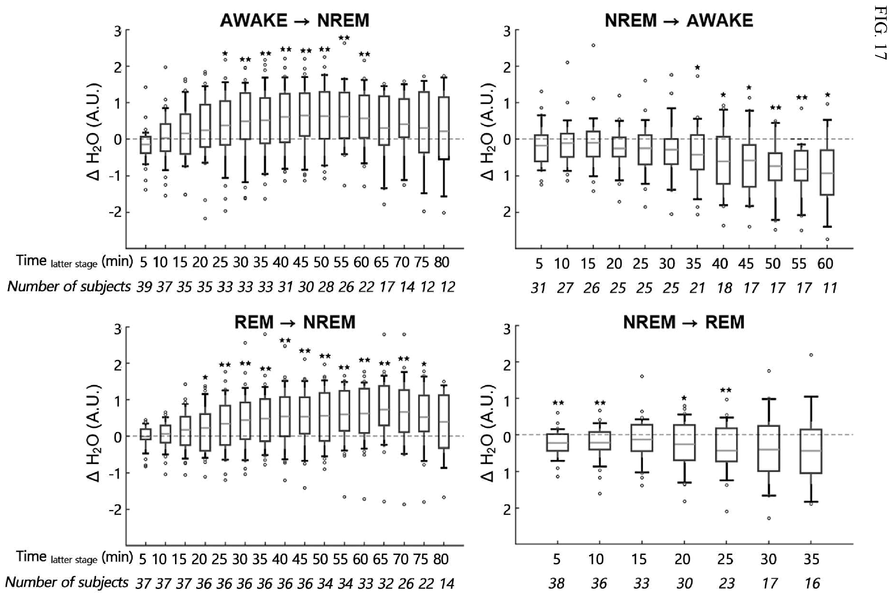

Table 2 summarizes the changes in water fraction according to various sleep transitions and their statistical significance. FIG. 17 shows a boxplot of water fraction change versus time quantized in every 5 minutes. The change in moisture fraction is calculated relative to the water fraction in the last 5 minutes of the previous sleep stage. The horizontal line within the box represents the median for each 5-minute segment and the boxes represents the 25th and 75th percentiles of the sample. Circles represent outliers located beyond 1.5 times the interquartile range.

In the AWAKE -> NREM transition, water content increased significantly after the sleep stage transition. During the first 45 minutes after sleep, the amount of water increased and then began to decrease. A significant decrease in water content was observed during the NREM -> AWAKE transition. The volume of water continued to decrease until the end of the experiment, 60 minutes after waking up.

Similar results were observed between REM -> NREM transition, showing a clear increase in water fraction during REM -> NREM transition and a clear decrease during NREM -> REM transition. In the REM -> NREM transition, the amount of water increases for 65 minutes after the sleep stage transition.

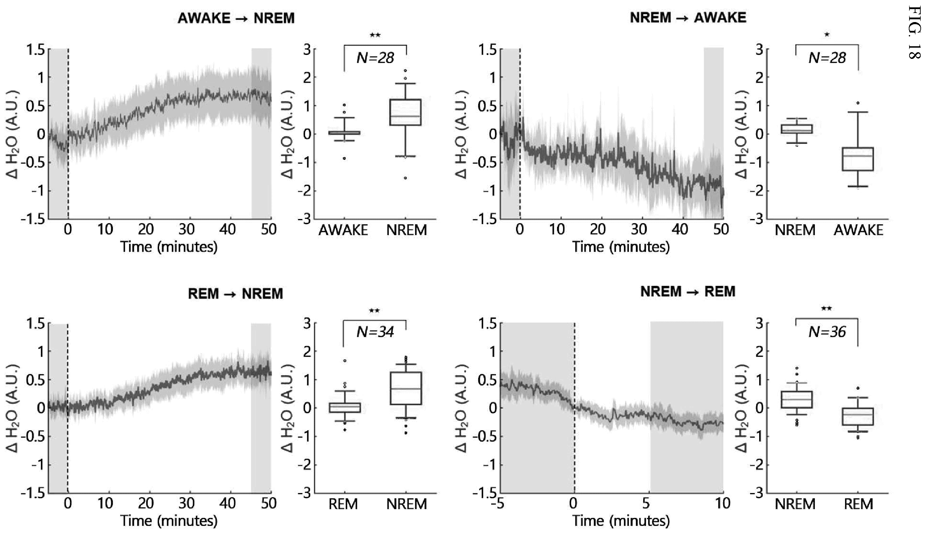

FIG. 18 shows the average water percentage after each sleep transition. The shaded interval represents the last 5-minute period of each sleep stage from which water fraction data is extracted for statistical analysis. Water fraction at water transition is zero-aligned to use as a baseline. The shaded area represents the 95% confidence interval.

In FIG. 18, the dashed line on the left represents the beginning of the sleep transition, and on the right is a boxplot of the water fraction in the last 5-minute segment of each sleep stage.

**[Table 2]**

| **T_{destination} (min)** | **AWAKE → NREM** | | | **NREM - AWAKE** | | |
|---|---|---|---|---|---|---|
| | **N** | **ΔH₂O (A.U.)** | **p** | **N** | **ΔH₂O (A.U.)** | **p** |
| **5** | 39 | -0.15 | 0.052 | 31 | -0.18 | 0.10 |
| **10** | 37 | 0.030 | 0.79 | 27 | -0.11 | 0.39 |
| **15** | 35 | 0.16 | 0.24 | 26 | -0.10 | 0.50 |
| **20** | 35 | 0.24 | 0.12 | 25 | -0.25 | 0.056 |
| **25** | 33 | 0.38 | <0.05 | 25 | -0.25 | 0.098 |
| **30** | 33 | 0.49 | < 0.01 | 25 | -0.29 | 0.096 |
| **35** | 33 | 0.52 | < 0.01 | 21 | -0.42 | < 0.05 |
| **40** | 31 | 0.61 | < 0.001 | 18 | -0.60 | < 0.05 |
| **45** | 30 | 0.65 | < 0.001 | 17 | -0.58 | < 0.05 |
| **50** | 28 | 0.63 | < 0.001 | 17 | -0.73 | < 0.01 |
| **55** | 26 | 0.62 | < 0.001 | 17 | -0.82 | < 0.001 |
| **60** | 22 | 0.57 | < 0.01 | 11 | -0.93 | < 0.05 |
| **65** | 17 | 0.31 | 0.24 | | | |
| **70** | 14 | 0.41 | 0.11 | - | | |
| **75** | 12 | 0.31 | 0.37 | | | |
| **80** | 12 | 0.22 | 0.54 | - | | |

| **T_{destination} (min)** | **REM → NREM** | | | **NREM → REM** | | |
|---|---|---|---|---|---|---|
| | **N** | **ΔH₂O (A.U.)** | **p** | **N** | **ΔH₂O (A.U.)** | **p** |
| **5** | 37 | -0.011 | 0.83 | 38 | -0.22 | < 0.01 |
| **10** | 37 | 0.062 | 0.34 | 36 | -0.21 | < 0.01 |
| **15** | 37 | 0.17 | 0.067 | 33 | -0.13 | 0.21 |
| **20** | 36 | 0.22 | < 0.05 | 30 | -0.26 | < 0.05 |
| **25** | 36 | 0.34 | < 0.01 | 23 | -0.42 | < 0.01 |
| **30** | 36 | 0.44 | < 0.01 | 17 | -0.40 | 0.13 |
| **35** | 36 | 0.48 | < 0.001 | 16 | -0.43 | 0.12 |
| **40** | 36 | 0.54 | < 0.001 | | | |
| **45** | 36 | 0.54 | < 0.001 | | | |
| **50** | 34 | 0.56 | < 0.001 | | | |
| **55** | 34 | 0.60 | < 0.001 | | | |
| **60** | 33 | 0.62 | < 0.001 | | | |
| **65** | 32 | 0.73 | < 0.001 | | | |
| **70** | 26 | 0.66 | < 0.01 | | | |
| **75** | 22 | 0.52 | < 0.05 | | | |

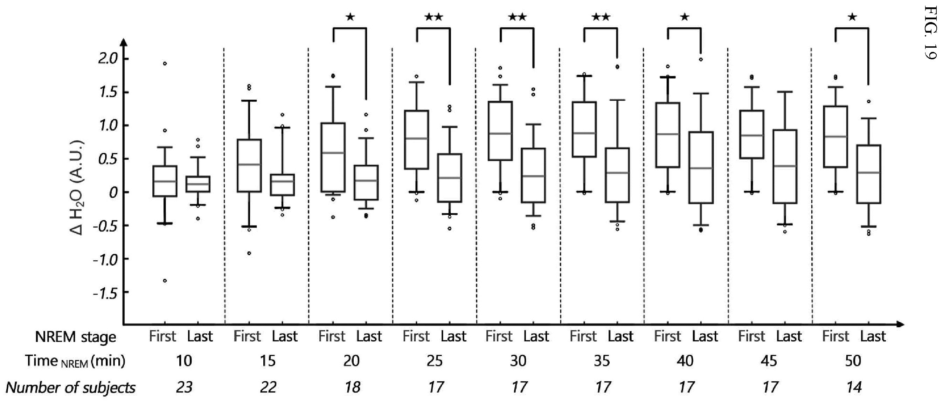

Additionally, the temporal response of water content during the NREM stage was observed to exhibit specific patterns during the sleep process. As shown in FIG. 19, the first NREM stage shows faster and larger water content changes compared to the last NREM stage (p<0.01). These results indicate that the glymphatic system is more active early in sleep.

FIG. 19 shows a comparison of water fraction changes between the first and last NREM stages. The Y-axis represents the change in water fraction over the first 5 minutes. Changes in water fraction are analyzed by increasing the duration of the NREM stage in 5 min increments. As the duration increases, the number of subjects decreases.

### Water content comparison between REM stages

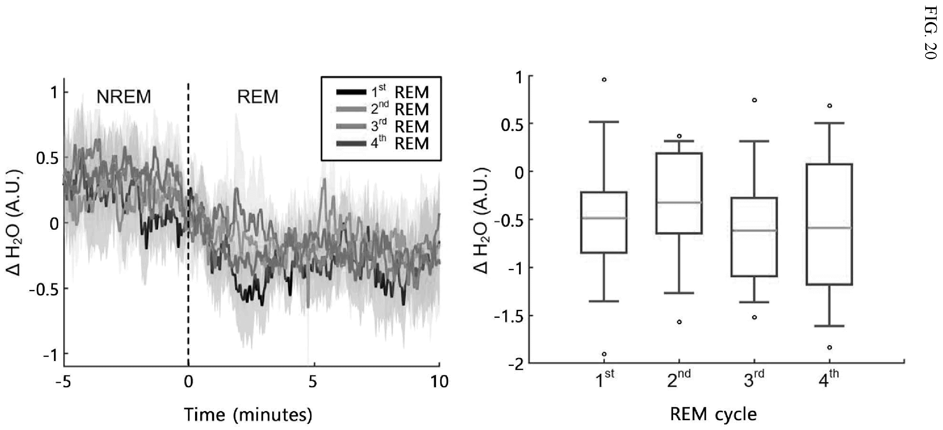

Statistical analysis of REM cycles was performed on 14 participants with four or more REM stage durations during the experiment. FIG. 20 shows the average water content for each REM stage. There is no significant difference in water changes between REM stages.

In FIG. 20, the y-axis represents the change between the average water content of the last 5 minutes of the source NREM stage and the average water content of the second 5-minute section of each REM stage.

### DISCUSSION

In this experiment, we evaluated the glymphatic system using near-infrared spectroscopy (NIRS), a non-invasive, continuous, real-time approach to detect water in brain tissue. We investigated changes in water fraction according to sleep stage transitions using a NIRS device using water-sensitive 925 nm wavelength light and PSG.

Non-parametric data analysis showed that signal changes (water fraction changes) are strongly dependent on the sleep-wake cycle, implying that clearance through the glymphatic system follows sleep-wake dynamics.

The main finding of this experiment is that the water content of brain tissue increased after the onset of sleep and decreased after awakening, and the water fraction significantly increased after the REM-NREM transition and decreased after the NREM-REM transition. Additionally, the water fraction during the first NREM cycle was significantly higher than during the last NREM cycle, but there was no difference in water fraction between different REM cycles.

### The concept of the glymphatic system in relation to water dynamics.

The glymphatic system is a macroscopic waste removal network through which CSF is exchanged with ISF, and utilizes the perivascular space (Virchow-Robin space), a unique complex of perivascular tunnels formed by viscous cells facilitated by AQP4 water channels. A key component of the glymphatic system is unidirectional water flow consisting of CSF and ISF. CSF, produced in the choroid plexus, is composed primarily of water (99%), with the remaining 1% composed of proteins, ions, neurotransmitters, and glucose. Chemically, ISF is considered similar in composition to CSF and is virtually indistinguishable. Therefore, the water fraction of brain tissue can represent a quantitative measure of water changes between CSF and ISF.

However, considering that the brain's fluid compartment consists of intracellular fluid (ICF) (60%-68%), ISF (12%-20%), blood (10%), and CSF (10%), it can be assumed that the measured water content of brain tissue is only a change between CSF and ISF. To maintain electrical neutrality and osmotic balance between the ICF and ECF, water flows into or out of the cell through the semipermeable cell membrane, resulting in changes in ICF volume.

As an osmotic pressure gradient is created by an impermeable solute such as Na+, osmotic water flow occurs and the ICF volume contracts or expands. Based on body fluid homeostasis while keeping the volume and composition of the ICF and ECF constant, ICF volume does not easily adapt to rapid changes and tends to be very stable. Additionally, cerebral blood flow (CBF) decreases after sleep onset and varies throughout the sleep-wake cycle. CBF decreases during NREM sleep, particularly by a further 25% in slow wave sleep (SWS), leading to a 10% decrease in cerebral blood volume (CBV), and increases during REM sleep. Previous studies have shown using NIRS that CBV in the prefrontal cortex increased from N2 sleep to SWS. Therefore, it is assumed that the increase in water volume of brain tissue during sleep is a result of CSF-ISF volume expansion and accelerates clearance by the glymphatic system.

### Dynamic changes in brain tissue water during the sleep-wake cycle.

The continuous filtration of toxins from the brain by the glymphatic system is regulated by the sleep-wake cycle, suppressed during wakefulness and significantly enhanced during sleep. Recent studies have shown that sleep is a major driver of the clearance the glymphatic system activity. Aβ42 levels in CSF decreased by 6% during unrestricted sleep, with Aβ42 levels in CSF being highest before sleep and lowest after awakening, but not during sleep deprivation. A difference of 75.8 pg/mL in Aβ levels was found between the unrestricted and sleep-deprived groups. Aβ burden in the hippocampus, one of the first brain regions to show neuropathological changes in Alzheimer's disease, was increased after sleep deprivation using F-florbetaben-PET. Several researchers have attempted to use non-invasive methods to evaluate and visualize the function and structure of the glymphatic system and its regulation by the sleep-wake cycle. Sleep is followed by an increase in whole brain CSF volume, quantified by voxel-based morphometric analysis, as well as a slow-visible diffusion coefficient in the cerebellum and left temporal pole, indicating changes in water diffusion and indirectly CSF volume.

Our results demonstrate the feasibility of assessing the glymphatic system with simple, non-invasive, continuous, and real-time measurements, and add to previous studies by demonstrating that the glymphatic system is regulated by the sleep-wake cycle. We found a strong association between sleep and glial lymphatic activity, showing that the water fraction steadily increased and decreased during sleep onset and approximately 1 hour after awakening. These findings support a hypothetical cascade in which sleep enhances glymphatic system Aβ clearance from the brain, and conversely, disrupted sleep increases the defective protein, ultimately increasing the risk of neurodegenerative diseases. The neuromodulator norepinephrine, a key driver of wakefulness, may be a key regulator of the activity of glymphatic system associated with the sleep-wake cycle. The decrease in plasma levels of norepinephrine associated with the onset of sleep leads to an expansion of the volume of the brain's interstitial space, which reduces tissue resistance to water flow and consequently allows CSF-ISF exchange. Additionally, changes in water fraction occur during the transition between NREM and REM sleep, similar to the pattern between wakefulness and sleep, suggesting that the glymphatic system is suppressed during REM sleep, which is similar to wakefulness in neurophysiological properties. Changes in water fraction associated with sleep stages may be dependent on the occurrence of slow wave activity (SWA), which exhibits spectral power densities in the range 0.75 - 4.5 Hz and is consistent with CSF-ISF exchange in the glymphatic system. Maximal levels of SWA are present in N3 sleep, but almost no SWA is present during REM sleep. In reality, SWA gradually increases during each NREM sleep and then rapidly decreases just before the onset of REM sleep. The dynamics of SWA may be closely related to changes in water fraction, which are thought to reflect the activity of glymphatic system.

Given that fewer NREM stages occur and N3 sleep becomes less pronounced as the night progresses, we reasoned that the activity of glymphatic system may gradually decrease throughout the night. As expected, we found that the water fraction during the first NREM cycle was higher than during the last NREM cycle, supporting the concept that NREM sleep is important for enhancing the activity of glymphatic system, which can be manipulated by achieving deep NREM sleep consecutively. The lower absolute amount of the last NREM sleep cycle together with the decrease in SWA over successive NREM cycles may contribute to this result. Unlike the change in water fraction according to the NREM cycle, no significant difference in water fraction was found between REM cycles. As discussed above, the absolutely small amount of SWA during REM sleep may not have sufficient force to cause significant differences in SWA, which is essential for the activity of glymphatic system during the REM cycle.

### Strengths and Limitations

This study has several key strengths. First, the custom-designed wireless NIRS device enabled continuous monitoring of water rates without disrupting the patient's sleep. Simultaneous measurement using the PSG device allowed the water content to be measured according to sleep stage transitions. Multimodal measurements showed an increase in water in the NREM stage and a decrease in water in the REM and AWAKE stages. Additionally, PSG testing allowed screening subjects with poor sleep quality, including OSA or PLM.

One limitation of our study was the absence of individual optical coefficients, namely scattering coefficient and absorption coefficient. In this study, statistical averages of human optical properties from previous studies are used. However, without accurate individual optical properties, measurement errors can occur and susceptibility to crosstalk artifacts can increase. Additionally, intermittent head and body movements can degrade signal integrity and make measurement data inappropriate for analysis. Therefore, this study was performed using only data sets that are not affected by motion artifacts, which hinders the investigation of the motion dependence of water content.

### CONCLUSION

The relationship between the human lymphatic system and sleep stages has been investigated. A custom-designed wireless NIRS device enabled measurement of glymphatic system activity related to sleep stages. Additional work related to precise individual optical coefficients can improve the overall truthfulness of the results.

### [Experimental Example 2]

Sleep deprivation or sleep diseases (sleep apnea, insomnia, etc.) result in a decrease in glymphatic system activity due to a decrease in sleep efficiency such as a decrease in deep sleep, an increase in light sleep, and sleep fragmentation, causing pathological waste deposition, which can lead to abnormal brain function or cranial nerve disease. In patients with sleep apnea, PiB-PET showed high amyloid beta deposition in the right temporal cortex and right posterior cingulate, which may be related to decreased glymphatic system activity. Glymphatic system activity can be monitored and quantitatively measured to confirm that glymphatic system activity is a determinant of sleep deprivation or sleep diseases. In particular, an increase in glymphatic system activity can be confirmed in insomnia patients who have received sleep intervention with insomnia cognitive behavioral therapy, neuromodulation, and drugs that increase the quantity or quality of sleep, and in sleep apnea patients who have received sleep intervention with continuous positive airway pressure treatment. That is, in patients with sleep deprivation or sleep diseases (sleep apnea, insomnia, etc.), through the dynamic changes in the activity of glymphatic system before and after treatment and tracking the treatment effect, the effect of sleep deprivation or sleep disease on the activity of glymphatic system as well as improvement of glymphatic system activity during appropriate treatment may be monitored and quantitatively measured non-invasively.

### [Experimental Example 3]

Mild cognitive impairment, a pre-stage of dementia, develops into dementia within 6 years in about 80% of cases, and without appropriate intervention, it progresses to serious dementia. Therefore, it is necessary to diagnose and evaluate mild cognitive impairment and prevent it from developing into irreversible dementia through active intervention. need. For early diagnosis of dementia, glymphatic system activity can be used as a method to evaluate cognitive decline before symptoms appear. Quantitative changes in glymphatic system activity in patients with mild cognitive impairment can be tracked and used as an effective target when treating and worsening mild cognitive impairment.

### [Experimental Example 4]

Factors that cause activation of the glymphatic system include various lifestyle habits such as exercise, drinking, sleeping posture, eating habits, and stress. In particular, increased exercise reduces amyloid beta deposition and improves cognitive function in Alzheimer's disease patients, which may be related to changes in glymphatic system activity. By non-invasively monitoring and quantitatively measuring the dynamic changes in glymphatic system activity before and after lifestyle habits correction, correctable glymphatic system activation factors can be identified. In addition, by confirming the increase in glymphatic system activity when correcting glymphatic system activation factors, it can be suggested that correction of glymphatic system activation factors is important for mental and physical health (cardiovascular disease, cerebrovascular disease, metabolic syndrome, dementia, etc.). Therefore, it is possible to promote glymphatic system activity by controlling the correctable lifestyle factors that determine glymphatic system activity, and at this time, monitoring and quantitative measurement of the dynamic changes in glymphatic system activity can be used as targets for treatment effectiveness.

### [Experimental Example 5]

The AQP4 channel is important for the rapid exchange between cerebrospinal fluid and interstitial fluid in the glymphatic system. In particular, the removal rate of amyloid beta decreases to 45% in AQP4 gene knockout mouse, and due to the correlation between Alzheimer's disease pathological diagnostic criteria and AQP4 distribution, changes in AQP4 expression and distribution have recently emerged as a pathological mechanism of Alzheimer's disease. Recently, there have been attempts to treat neurological diseases such as neuromyelitis optica and multiple sclerosis by controlling AQP4 expression and function using metal ions and molecular inhibitors. Inspired by this, when AQP4 function is improved, the activity of glymphatic system can increase, and by monitoring and quantitatively measuring the improvement in glymphatic system activity with it, the treatment prognosis of Alzheimer's disease can be examined.

Above, various preferred embodiments of the present invention have been described by giving some examples, but the description of the various embodiments described in the "Detailed Contents for Carrying out the Invention" section is merely illustrative, and those skilled in the art will understand well from the above description that the present invention can be implemented with various modifications or equivalent implementations of the present invention.

In addition, since the present invention can be implemented in various other forms, the present invention is not limited by the above description. The above description is intended to make the disclosure of the present invention complete and is provided only to fully inform those skilled in the art of the present invention of the scope of the present invention. It should be noted that the present invention is only defined by each claim in the claims.

### [Explanation of symbols]

110. Near-infrared spectroscopy unit,
120. Data processing unit,
130. First data processing unit,
140. Second data processing unit,
150. Third data processing unit,
160. Fourth data processing unit,
170. Database,
180. Fifth data processing unit,
190. Sixth Data Processing unit,
195. Control unit

## Claims

1. A method for monitoring brain water based on near-infrared spectroscopy, performed by a processor, the method comprising:
calculating oxidized hemoglobin, reduced hemoglobin, and water fraction based on signals measured by the near-infrared spectroscopy;
estimating a hemoglobin-correlated component included in the water fraction by using linear estimation based on a correlation between the water fraction and a hemoglobin signal;
extracting a pure water fraction from the water fraction by removing the estimated hemoglobin-correlated component; and
outputting the extracted pure water fraction.

2. The method according to claim 1, further comprising:
removing artifacts from the signals measured by the near-infrared spectroscopy,
wherein the removing of the artifacts is performed before or after the calculating.

3. The method according to claim 2,
wherein the removing of the artifacts is **characterized by**:
removing, in a preprocessing process, non-ideal contamination signals caused by at least one of heart rate, respiration, pulse, neural activity, or vascular movement by low-pass filtering the signals measured by the near-infrared spectroscopy, and removing, in a post-processing process, motion artifacts through signal restoration using spline interpolation.

4. The method according to claim 1,
wherein the estimating of the hemoglobin-correlated component is **characterized by**:
modeling the calculated water fraction as a sum of a target water component, a first hemoglobin-correlated component corresponding to the oxidized hemoglobin, and a second hemoglobin-correlated component corresponding to the reduced hemoglobin, and
estimating the first hemoglobin-correlated component and the second hemoglobin-correlated component as the hemoglobin-correlated component included in the water fraction.

5. The method according to claim 4,
wherein the extracting is **characterized by**:
extracting the pure water fraction by minimizing the first hemoglobin-correlated component and the second hemoglobin-correlated component while leaving only the target water component, using ordinary least squares (OLS) regression in which the oxidized hemoglobin and the reduced hemoglobin are regressors and the water fraction is a dependent variable.

6. The method according to claim 5,
wherein the extracting is **characterized by**:
extracting the pure water fraction by further minimizing crosstalk artifacts by applying linear minimum mean square error (LMMSE).

7. The method according to claim 1,
wherein the estimating comprises:
estimating, from the signals measured by the near-infrared spectroscopy, a coefficient relating the hemoglobin signal to the water fraction, the hemoglobin-correlated component being a product of the coefficient and the hemoglobin signal.

8. The method according to claim 1, further comprising:
measuring glymphatic system activity based on the extracted pure water fraction, and providing information on the measured glymphatic system activity.

9. The method according to claim 1, further comprising:
constructing standard data of brain tissue water based on measurements taken in a selected group; and
providing evaluation information by evaluating glymphatic system activity based on the extracted pure water fraction and the standard data of brain tissue water,
wherein the providing is **characterized by**:
comparing the extracted pure water fraction with the standard data of brain tissue water, and, when a significant difference from the standard data of brain tissue water occurs, providing the difference by scoring the difference as a categorization result or a standardized value corresponding to the difference.

10. The method according to claim 1, further comprising:
constructing standard data of brain tissue water based on measurements taken in a selected group; and
providing information on a sleep state according to glymphatic system activity for wakefulness-sleep or for each sleep stage, based on the extracted pure water fraction and the standard data of brain tissue water,
wherein the providing is **characterized by**:
comparing the extracted pure water fraction with the standard data of brain tissue water, and, when a significant difference from the standard data of brain tissue water occurs, providing the information on the sleep state according to the glymphatic system activity by scoring the difference as a categorization result or a standardized value corresponding to the difference; or
providing the information on the sleep state according to the glymphatic system activity by monitoring changes in water fraction according to sleep stage transitions based on the extracted pure water fraction.

11. The method according to claim 1, further comprising:
providing information on cerebral edema and cerebral pressure by measuring the cerebral edema and the cerebral pressure based on the extracted pure water fraction,
wherein the providing is **characterized by**:
measuring an amount of brain tissue water at regular intervals, comparing the measured amount with an initial value, and providing the information on the cerebral edema and the cerebral pressure by scoring a difference between the measured amount and the initial value as a categorization result or a standardized value corresponding to the difference.

12. The method according to claim 11, further comprising:
controlling a cerebral pressure regulator based on the information on the cerebral pressure measured in the providing.

13. A device for monitoring brain water based on near-infrared spectroscopy, comprising:
a near-infrared spectrometer unit (110) that outputs a near-infrared signal to a subject and receives a signal reflected from the subject; and
a data processing unit (120) for processing the signal obtained from the near-infrared spectrometer unit (110),
wherein the data processing unit (120) is configured to monitor brain water changes by performing the method according to claim 1.

14. The device according to claim 13, further comprising:
a database (170) storing neurodegenerative brain disease indicators corresponding to glymphatic system activity,
wherein the data processing unit (120) is configured to:
measure glymphatic system activity based on the pure water fraction extracted when monitoring the brain water changes, search the database (170) for a neurodegenerative brain disease indicator corresponding to the measured glymphatic system activity, and then provide auxiliary information related to prediction, progression, diagnosis, and treatment of neurodegenerative brain disease based on the searched indicator.

15. The device according to claim 13,
wherein the data processing unit (120) is configured to:
measure cerebral edema and cerebral pressure based on the pure water fraction extracted when monitoring the brain water changes, and provide information on the measured cerebral edema and cerebral pressure.

16. The device according to claim 15, further comprising:
a control unit (195) configured to control a cerebral pressure regulator based on the cerebral pressure output from the data processing unit (120).
